# EUROPEAN PATENT SPECIFICATION

(11) **EP 3 004 433 B1**
(45) Date of publication and mention of the grant of the patent: **05.09.2018**
(21) Application number: 14804039.7
(22) Date of filing: 28.05.2014
(51) Int. Cl.: C40B 40/06

(54) **SUBSTANTIALLY UNBIASED AMPLIFICATION OF GENOMES**
IM WESENTLICHEN GLEICHMÄSSIGE AMPLIFIZIERUNG VON GENOMEN
AMPLIFICATION PRATIQUEMENT NON BIAISÉE DE GÉNOMES

(30) Priority: 30.05.2013 US 201361829193 P
(43) Date of publication of application: 13.04.2016
(73) Proprietor: The Regents of the University of California, Oakland, CA 94607 (US)
(72) Inventor: GOLE, Jeffrey, San Diego, CA 92122 (US); ZHANG, Kun, San Diego, CA 92128 (US)
(74) Representative: Hutter, Anton
(86) International application number: PCT/US2014/039830
(87) International publication number: WO 2014/193980

(56) References cited:
- US-A1- 2006 014 167
- US-A1- 2006 068 394
- ISHOEY T ET AL: "Genomic sequencing of single microbial cells from environmental samples", CURRENT OPINION IN MICROBIOLOGY, CURRENT BIOLOGY LTD, GB, vol. 11, no. 3, 1 June 2008 (2008-06-01), pages 198-204, XP022797213, ISSN: 1369-5274, DOI: 10.1016/J.MIB.2008.05.006 [retrieved on 2008-06-10]
- LASKEN ET AL: "Single-cell genomic sequencing using Multiple Displacement Amplification", CURRENT OPINION IN MICROBIOLOGY, CURRENT BIOLOGY LTD, GB, vol. 10, no. 5, 17 October 2007 (2007-10-17), pages 510-516, XP022338248, ISSN: 1369-5274, DOI: 10.1016/J.MIB.2007.08.005
- JEFF GOLE ET AL: "Massively parallel polymerase cloning and genome sequencing of single cells using nanoliter microwells", NATURE BIOTECHNOLOGY, vol. 31, no. 12, 10 November 2013 (2013-11-10), pages 1126-1132, XP055318727, US ISSN: 1087-0156, DOI: 10.1038/nbt.2720
- MARCY ET AL.: 'Nanoliter Reactors Improve Multiple Displacement Amplification of Genomes from Single Cells' PLOS GENETICS; vol. 3, 01 September 2007, pages 1702 - 1708, XP002561035
- TANG ET AL.: 'mRNA-Seq whole-transcriptome analysis of a single cell' NATURE METHODS vol. 6, 19 April 2009, pages 377 - 384, XP055037482
- HUANG ET AL.: 'Non-biased and efficient global amplification of a single- cell cDNA library.' NUCL ACIDS RES 18 October 2013, pages 1 - 11, XP055297049

## Description

Embodiments herein relate generally to whole-genome amplification. Some embodiments herein related generally to unbiased amplification of a genome.

The genetic material in a single cell can be amplified by DNA polymerase into many clonal copies through whole genome amplification and characterized by shotgun sequencing. Single-cell genome sequencing has been successfully demonstrated on microbial and mammalian cells¹⁻⁶, and applied to the characterization of microbial genomic diversity of the ocean⁷, somatic mutations in cancers^{8,9}, and meiotic recombination and mutation in sperm^{3,10}.

Lasken ("Single-cell genomic sequencing using Multiple Displacement Amplification", Current Opinion in Microbiology, Current Biology Ltd, GB, vol. 10, no. 5, 17 October 2007, pages 510-516) discusses how single microbial cells can be sequenced using DNA amplified by the Multiple Displacement Amplication (MDA) reaction.

Ishoey et al. ("Genomic sequencing of single microbial cells from environmental samples", Current Opinion in Microbiology, Current Biology Ltd, GB, vol. 11, no. 3, 1 June 2008, pages 198-204) focuses on research strategies for putting the recently developed techniques of Laskin into practice in the laboratory setting.

### SUMMARY

In accordance with a first aspect of the invention, there is provided a method of producing a substantially unbiased amplification library of a genome of a single cell, the method comprising:
- applying a liquid comprising diluted whole cells or fractions thereof and the single cell to a substrate comprising a plurality of nanolitre-scale reaction environments each having a volume of 1 nanolitre to 50 nanolitres, wherein the single cell is in a nanolitre-scale reaction environment of the plurality of reaction environments, and wherein at least 95% of the nanolitre-scale reaction environments do not comprise any genomes other than the genome of the single cell;
- amplifying, by multiple strand displacement amplification, the genome of the single cell in the nanolitre-scale reaction environment, wherein a ratio of amount of nucleic acid of the genome to volume of the nanolitre-scale reaction environment is at least 0.3 Mega-basepairs per nanolitre, and wherein the nanolitre-scale reaction environment is configured for substantially unbiased amplification of the genome, said amplifying comprising contacting the reaction environment with (a) strand-displacement polymerase, and (b) a plurality of random multimers of DNA, wherein said amplifying is performed until additional cycles of amplification are no longer in a logarithmic phase, thereby producing a plurality of amplicons of a substantially unbiased amplification of the genome; and
- constructing a library comprising a plurality of amplicons of the substantially unbiased amplification of the genome.

In some embodiments, a ratio of amount of nucleic acid of the genome to volume of the nanolitre-scale reaction environment is at least 200 Mega-basepairs per nanolitre. In some embodiments, the nanolitre-scale reaction environment is configured for amplification of at least 90% of the genome at greater than 1x coverage. In some embodiments, the nanolitre-scale reaction environment comprises a volume of no more than 20nL. In some embodiments, the nanolitre-scale reaction environment comprises a volume of no more than 12nL. In some embodiments, the method further comprises amplifying a plurality of genomes of single cells in a plurality of nanolitre-scale reaction environments on a single substrate, wherein at least 95% of the reaction environments do not comprise any genomes other than a genome of a single cell. In some embodiments, at least 99% of the reaction environments do not comprise any genomes other than a genome of a single cell. In some embodiments, the substrate is configured for a single pipetting action to distribute the genomes of single cells among the reaction environments. In some embodiments, the method further comprises selecting a desired number of reaction environments; and amplifying the plurality of genomes of single cells in only the desired number of reaction environments. In some embodiments, the method further comprises identifying a reaction environment in which a desired level of amplification has been achieved, wherein the library is constructed from the reaction environment in which a desired level of amplification has been achieved. In some embodiments, the method further comprises constructing a plurality of libraries from the plurality of reaction environments, in which the number of the plurality of libraries is the same or different as the number of the plurality of reaction environments. In some embodiments, amplifying the genome of the single cell in the nanolitre-scale reaction environment comprises amplification in the presence of an amplification-detection moiety. In some embodiments, the amplification-detection moiety comprises a cyanine dye. In some embodiments, the amplification-detection moiety comprises SYBR™ green dye. In some embodiments, signal from the amplification-detection moiety identifies a reaction environment in which a desired level of amplification has been achieved. In some embodiments, the reaction environment does not comprise any cells other than the single cell. In some embodiments, the reaction environment does not comprise any genomes other than the genome of the single cell. In some embodiments, the random multimers are selected from the group consisting of: pentamers, hexamers, heptamers, octamers, nonamers and decamers. In some embodiments, the random multimers are hexamers. In some embodiments, substantially all of the plurality of amplicons are unbranched. In some embodiments, the method further comprises removing at least some of the plurality of amplicons from the reaction environment prior to constructing the library. In some embodiments, removing at least some of the plurality of amplicons comprises micromanipulation. In some embodiments, the plurality of amplicons comprises no more than t 100 picograms to 10 nanograms of DNA. In some embodiments, the library comprises a transposase-based library. In some embodiments, the library comprises a Tn5 transposase-based library. In some embodiments, the library comprises a random fragmentation and ligation library. In some embodiments, the single cell is one of a human cell or a microbial cell. In some embodiments, the single cell comprises a cell of a bacterium that is unculturable, or substantially unculturable. In some embodiments, the MDA comprises real time MDA. In some embodiments, the method is performed in parallel on two or more genomes of two or more single cells, thereby producing two or more unbiased amplification libraries in parallel. In some embodiments, the method further comprises at least one of: de novo assembly of unculturable bacteria in the human gut, de novo assembly of unculturable bacteria in heterogeneous environments such as sea water, copy number variation calling on single neurons, copy number variation calling on single cancerous cells or circulating tumor cells, or human haplotyping. In some embodiments, the strand-displacement polymerase comprises a high-fidelity polymerase. In some embodiments, the strand-displacement polymerase comprises phi29 polymerase.

In accordance with a second aspect, there is provided a method of producing a substantially unbiased amplification of a genome by multiple strand displacement amplification (MDA), the method comprising
- applying a liquid comprising diluted whole cells or fractions thereof to a substrate comprising a nanolitre-scale reaction environment having a volume of 1 nanolitre to 50 nanolitres, wherein there is at least a 99% probability that the nanolitre-scale reaction environment comprises no more than one genome, wherein a ratio of amount of nucleic acid of the genome to volume of the nanolitre-scale reaction environment is at least 0.3 Mega-basepairs per nanolitre; and
- contacting the nanolitre-scale reaction environment with (a) strand-displacement polymerase, and (b) a plurality of random multimers of DNA, and amplifying the genome by MDA until additional cycles of amplification are no longer in a logarithmic phase, thereby producing a substantially unbiased amplification of the genome.

In some embodiments, the method further comprises constructing a library comprising a plurality of amplicons of the substantially unbiased amplification of the genome. In some embodiments, the nanolitre-scale reaction environment is configured for amplification of at least 90% of the genome at greater than 1x coverage. In some embodiments, a ratio of amount of nucleic acid of the genome to volume of the reaction environment is at least 200 Mega-basepairs per nanolitre. In some embodiments, the random multimers are selected from the group consisting of: pentamers, hexamers, heptamers, octamers, nonamers, and decamers. In some embodiments, the random multimers comprise hexamers. In some embodiments, substantially all of the plurality of amplicons are unbranched. In some embodiments, the nanolitre-scale reaction environment comprises a nanolitre-scale reaction environment that facilitates substantially unbiased amplification of the single cells. In some embodiments, the nanolitre-scale reaction environment comprises a volume of no more than 20nL. In some embodiments, the nanolitre-scale reaction environment comprises a volume of no more than 12nL. In some embodiments, the method further comprises at least one of: de novo assembly of a genome of an unculturable bacterium of the human gut, de novo assembly of an unculturable bacterium of a heterogeneous environment, copy number variation calling on a single neuron, copy number variation calling on a single cancerous cell or circulating tumor cell, or human haplotyping. In some embodiments, the strand-displacement polymerase comprises a high-fidelity polymerase. In some embodiments, the strand-displacement polymerase comprises phi29 polymerase.

The disclosure also provides a substrate for substantially unbiased amplification a genome at least one single cell is provided. The substrate can comprise a plurality of loading areas, in which each loading area is configured to receive a liquid sample. Each loading area can comprise a plurality of nanolitre-scale reaction environments that facilitates substantially unbiased amplification of a single cell. The plurality of nanolitre-scale reaction environments may be configured for performing a desired number of amplification reactions in parallel, in which each amplification reaction is conducted in a different nanolitre-scale reaction environment. Preferably, the plurality of nanolitre-scale reaction environments is configured for performing a desired number of amplification reactions in parallel without further modification of the substrate. Preferably, the plurality of nanolitre-scale reaction environments are not in fluid communication with any microfluidic channels or nanofluidic channels. Preferably, each nanolitre-scale reaction environment has a volume of no more than 12 nL. Preferably, each nanolitre-scale reaction environment has a volume of no more than 20 nL. Preferably, each loading area is configured for loading a solution comprising diluted cells into the plurality of nanolitre-scale reaction environments via a single pipetting action. Preferably, each reaction environment comprises a plurality of random multimers and strand-displacement polymerase. Preferably, the plurality of multimers comprises hexamers. Preferably, the substrate comprises at least three loading areas. Preferably, each loading area comprises at least ten nanolitre-scale reaction environments. Preferably, each loading area comprises at least one hundred nanolitre-scale reaction environments. Preferably, the substrate further comprises a detector configured to detect an amplification-detection moiety in each of the reaction environments. Preferably, the substrate further comprises a nanopipettor configured to withdraw amplified nucleic acid from a single reaction environment. Preferably, the nanolitre-scale reaction environments are configured so that at least 99% of the reaction environments comprise a genome of no more than one cell following a loading of solution comprising single cells or fractions thereof in the loading area. Preferably, substantially each reaction environment comprises a genome of no more than one cell, and wherein substantially each reaction environment that comprises a genome further comprises a plurality of amplicons of the genome. Preferably, the plurality of amplicons comprises substantially unbiased coverage of the genome. Preferably, the plurality of amplicons comprises no more than 100 picograms to 10 nanograms of DNA. Preferably, the strand-displacement polymerase comprises a high-fidelity polymerase. Preferably, the strand-displacement polymerase comprises phi29 polymerase.

### BRIEF DESCRIPTION OF THE DRAWINGS

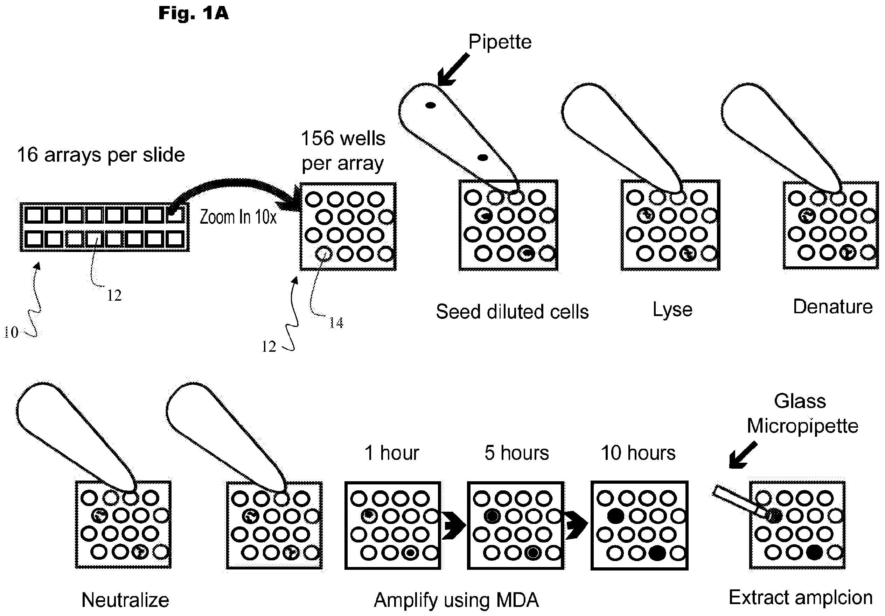

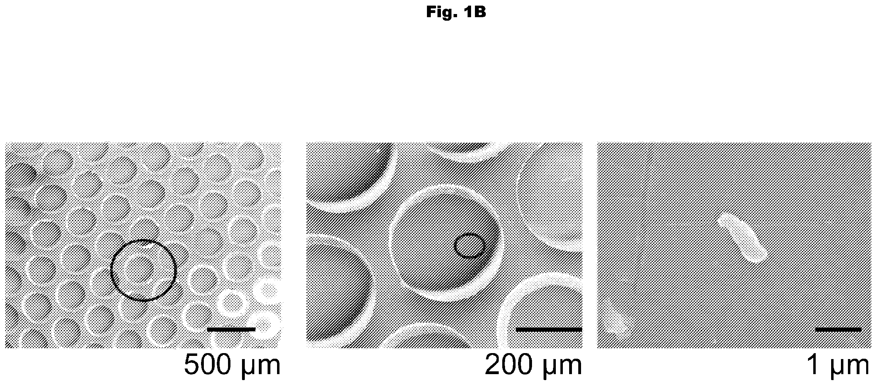

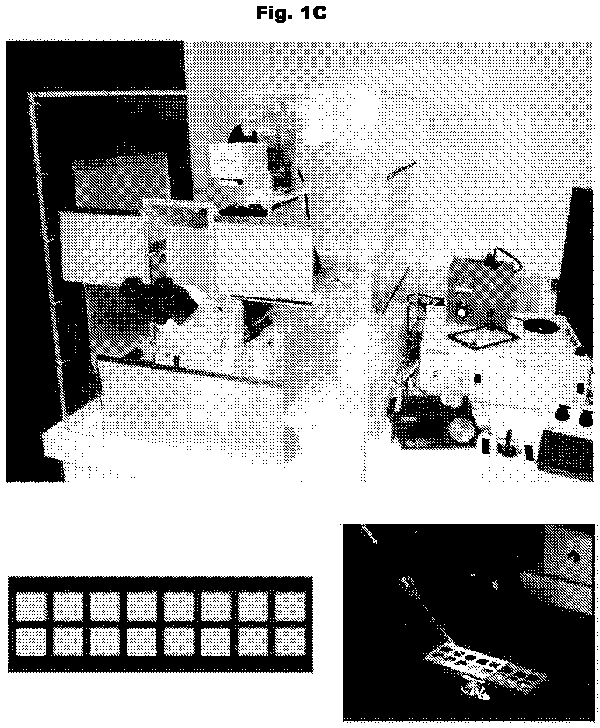

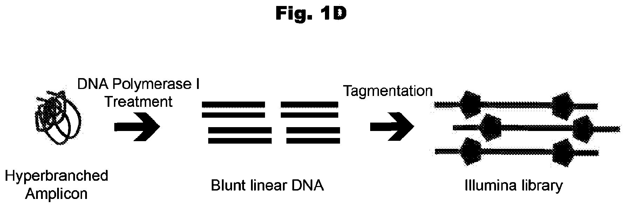

**Figure 1** is a series of schematic diagrams illustrating substantially unbiased amplification of genomes. **Figure 1A** is a schematic diagram illustrating a substrate **100** in the context of a method of substantially unbiased amplification of genomes. Each substrate **100** can contain 16 individual loading areas **12,** with each loading area **14** containing 255 nanolitre-scale reaction environments, for example 12nl microwells. Cells, lysis solution, denaturing buffer, neutralization buffer, and MDA master mix comprising an amplification-detection moiety can be each added to the microwells with a single pipette pump. Amplicon growth can be then visualized with a fluorescent microscope using a real time MDA system. Microwells showing increasing fluorescence over time are positive amplicons. The amplicons are extracted with fine, glass pipettes attached to a micromanipulation system. **Figure 1B** is a series of scanning electron microscopy (SEM) images of a single *E. coli* cell at different magnifications. This particular well contains only 1 cell, and most wells observed also contained no more than 1 cell. **Figure 1C** is a photograph illustrating a custom microscope incubation chamber that can be used for real time MDA. The chamber is temperature and humidity controlled to mitigate evaporation of reagents. Additionally, it prevents contamination during amplicon extraction by self-containing the micromanipulation system. An image of the entire microwell array is also shown, as well as a micropipette probing a well. **Figure 1D** is a schematic diagram illustrating complex 3-dimensional MDA amplicons are reduced to linear DNA using DNA polymerase I and Ampligase. This process can significantly improve the library complexity post-tagmentation.

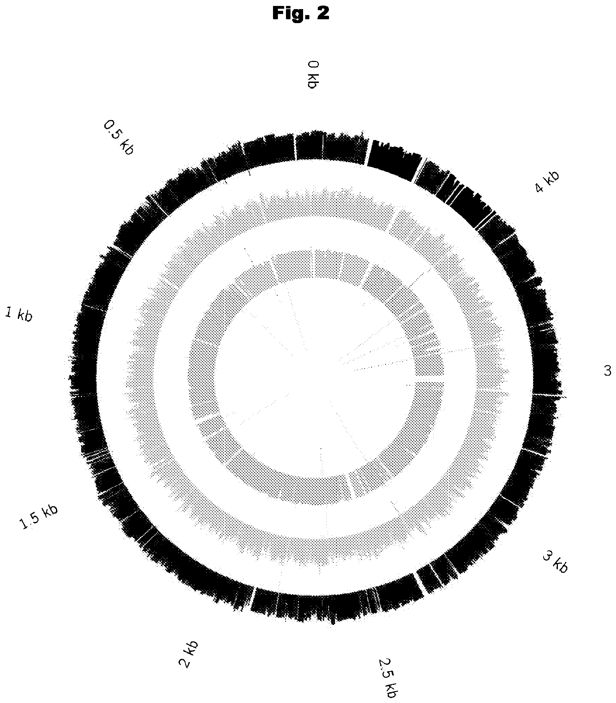

**Figure 2** is a diagram of assembled *E. coli* genomes generated by MIDAS. Three single *E. coli* cells were analyzed using MIDAS. Between 88% and 94% of the genome was assembled with very little sequencing effort (2-8M PE100bp reads). The histograms show the log₂ of average depth of coverage across each assembled region for each of the three cells. Gaps are represented by blank whitespace in between color contigs. Depth of coverage is fairly uniform across the genome, and few gaps are present.

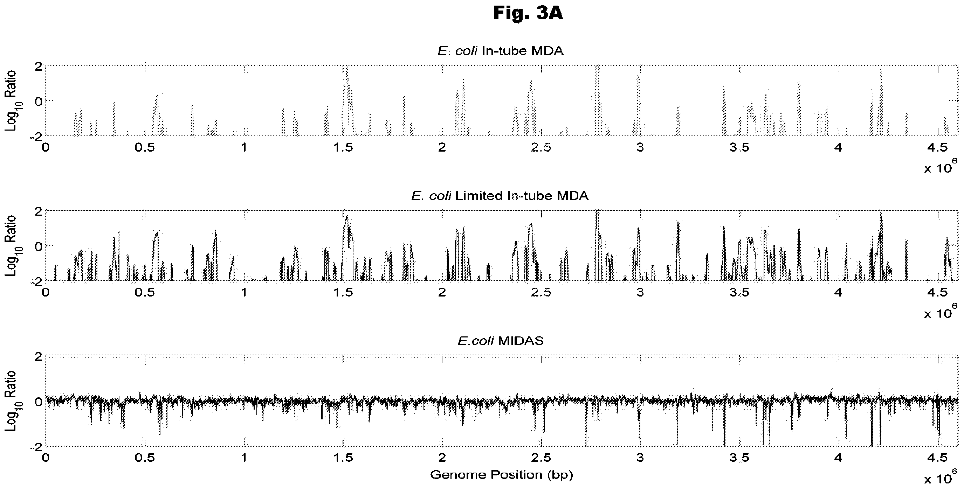

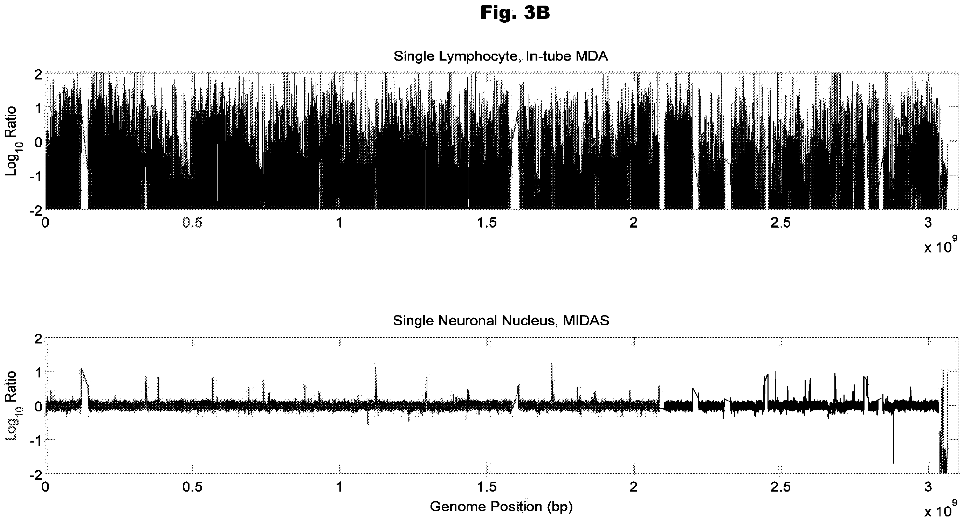

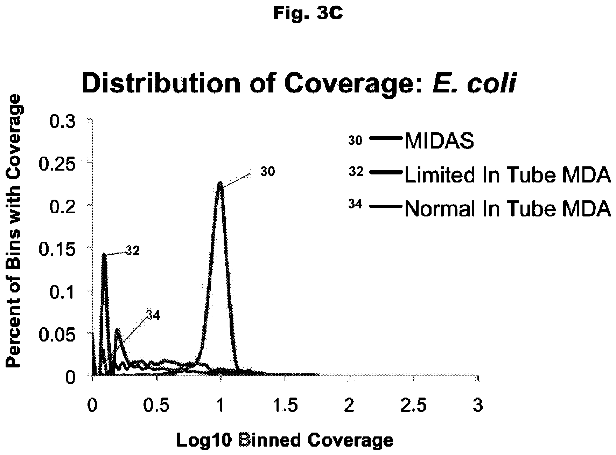

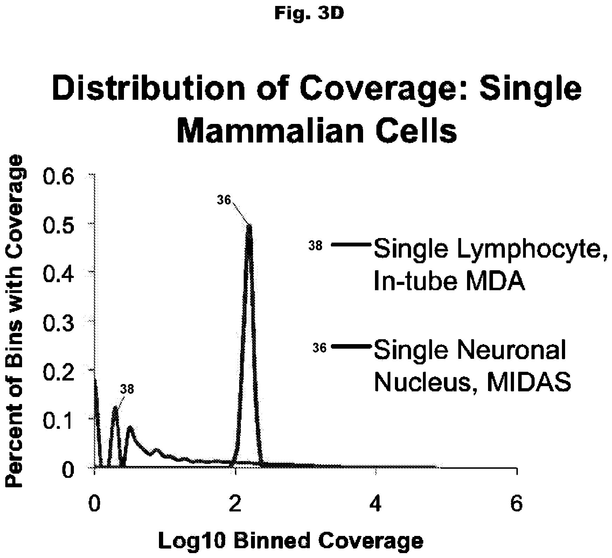

**Figure 3** is a series of graphs illustrating genomic coverage of single bacterial and mammalian cells post MDA and MIDAS. **Figure 3A** is a graph illustrating a comparison of single *E. coli* cells amplified in a PCR tube for 10 hours (top), 2 hours (middle), and in a microwell (MIDAS) for 10 hours (bottom). Log₁₀ ratio (y-axis) represents the normalized coverage. The bias improves as MDA is limited, with the MIDAS method displaying the greatest uniformity. **Figure 3B** is a graph illustrating a comparison of single human cells amplified using traditional MDA and MIDAS. A 10 hour MDA of a single lymphocyte (top) displays more coverage bias when compared to a single neuronal nucleus amplified by MIDAS (bottom). **Figure 3C** is a graph illustrating distribution of coverage of amplified single bacterial cells. The x-axis represents the log₁₀ of genomic coverage binned into 100 total bins. MIDAS (**30**) demonstrates a tight coverage, indicating limited bias in the library. Both the normal (**32**) and limited (**34**) in-tube MDA libraries show a broad range of coverages. **Figure 3D** is a graph illustrating distribution of coverage of amplified single mammalian cells. MIDAS (**36**) shows a much tighter coverage distribution than an in-tube MDA library (**38**).

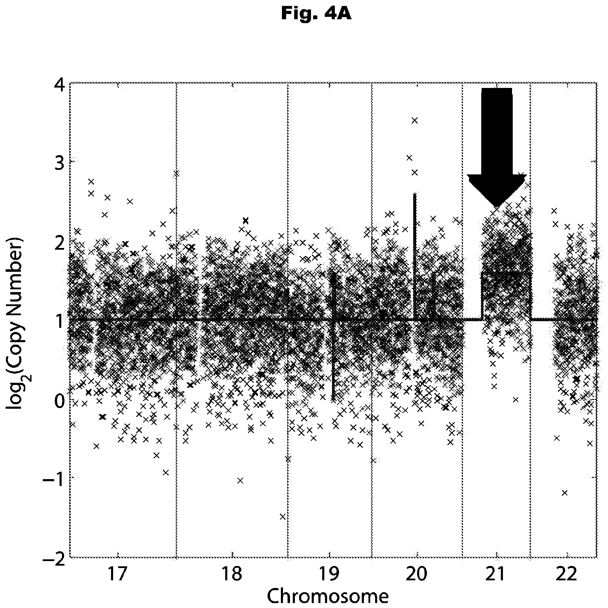

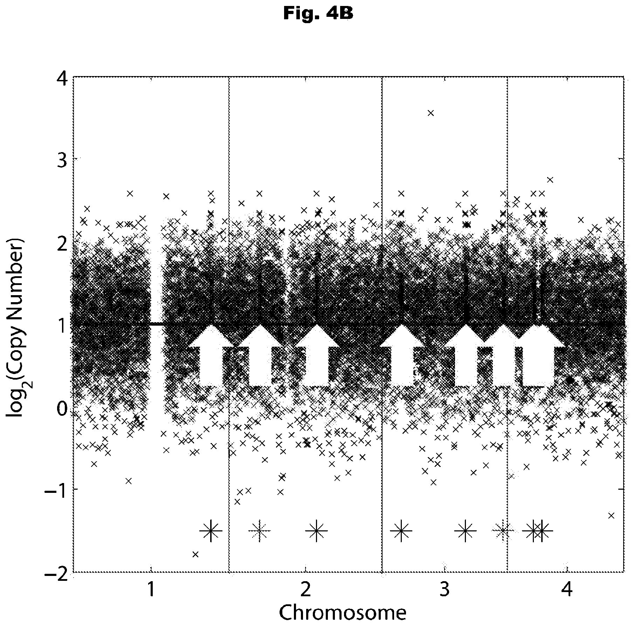

**Figure 4** is a series of graphs illustrating detection of copy number variants using MIDAS. **Figure 4A** is a graph illustrating a plot of copy number variation in a Down Syndrome single cell analyzed with MIDAS. The x-axis shows genomic position, while the y-axis shows (in a log₂ scale) the estimated copy number. Trisomy 21 is clearly visible in this single cell, along with several other smaller CNV calls. **Figure 4B** is a plot of copy number variation in a Down Syndrome single cell with Trisomy 21 "spike-ins". The x-axis shows genomic position, while the y-axis shows (in a log₂ scale) the estimated copy number. At each arrow, a 2 Mb section of chromosome 21 was computationally inserted into the genome. At each location, a copy number variant is called, showing that MIDAS can detect 2 Mb copy number variation accurately.

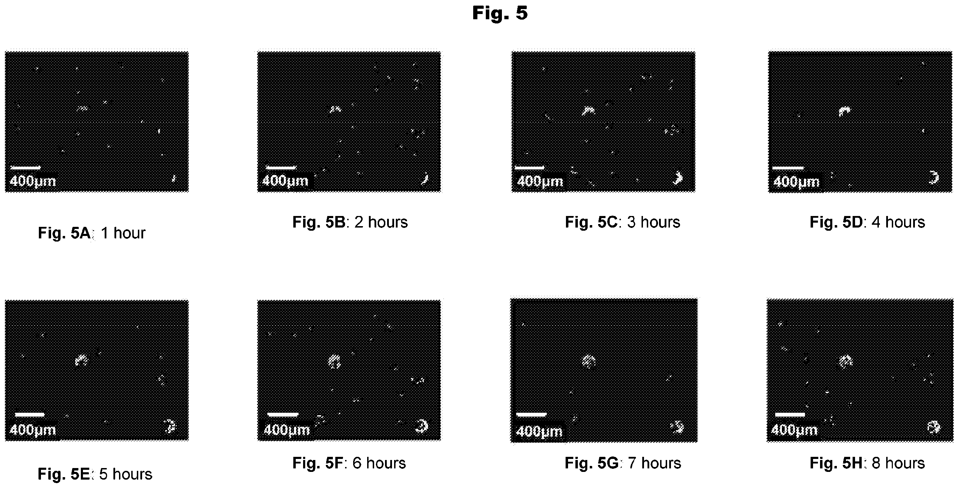

**Figure 5** is a series of microscope images depicting real time MDA. Images are taken every hour using a 488 nm filter. Shown are 1 hour **(Figu**r**e 5A**), 2 hours (**Figure 5B**), 3 hours (**Figure 5C**), 4 hours (**Figure 5D**), 5 hours (**Figure 5E**), 6 hours (**Figure 5F**), 7 hours (**Figure 5G**), and 8 hours (**Figure 5H**). Amplicons are visualized growing beginning at 1 hour and continue to grow until they cannot amplify due to the limited space in the microwells. This saturation usually occurs within 5 to 6 hours. The amplicons are randomly distributed demonstrating random cell seeding, and no amplicons are in abutting wells.

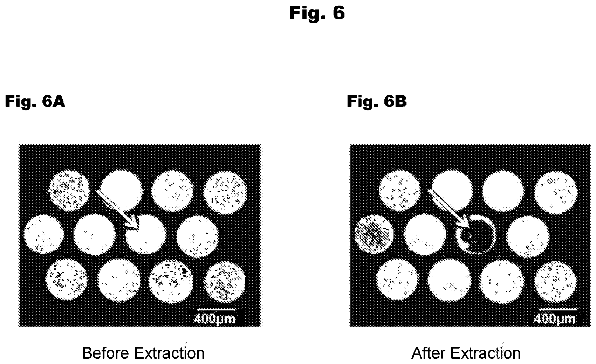

**Figure 6** is a series of microscope images depicting amplicon extraction. Microwells are saturated with genomic DNA and MDA is performed such that every well contains an MDA amplicon. The fluorescence in **Figure 6A** displays successful amplification. After amplification, a micropipette is lowered into a single well, designated by the arrow, and the amplicon is extracted. **Figure 6B** shows a successful removal of the amplicon due to loss of fluorescence, without disturbing the contents of the nearby microwells.

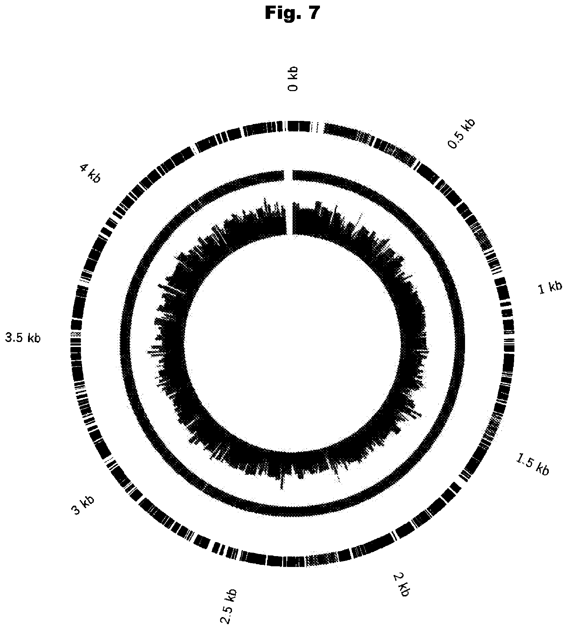

**Figure 7** is a schematic diagram depicting a comparison of assembly to mapped reads across a genome. The outer track displays the assembled contigs mapping to E. coli. The middle track shows the raw reads mapping to E. coli. The inner track presents the coverage of the reads. The coverage is less in the mapped regions where contigs were not assembled.

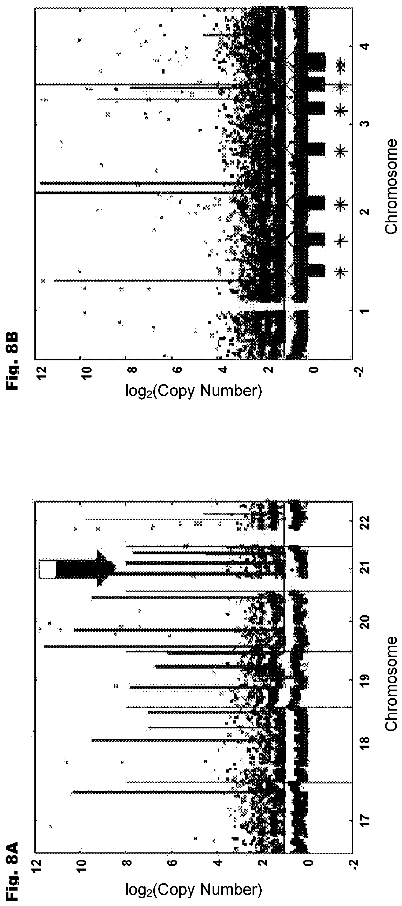

**Figure 8** is a series of graphs depicting detection of copy number variants using traditional MDA-based single cell sequencing. **Figure 8A** is a graph depicting a plot of copy number variation in a Down Syndrome single cell analyzed with traditional MDA. The x-axis shows genomic position, while the y-axis shows (in a log2 scale) the estimated copy number. Trisomy 21 is not visible in this single cell, and several other large CNVs spread across the genome are called. **Figure 8B** is a graph depicting a plot of copy number variation in a Down Syndrome single cell with Trisomy 21 "spike-ins." The x-axis shows genomic position, while the y-axis shows (in a log2 scale) the estimated copy number. At each arrow, a 2 Mb section of chromosome 21 was computationally inserted into the genome. Copy number variation is not called at any location, showing that traditional MDA-based methods cannot detect CNVs accurately.

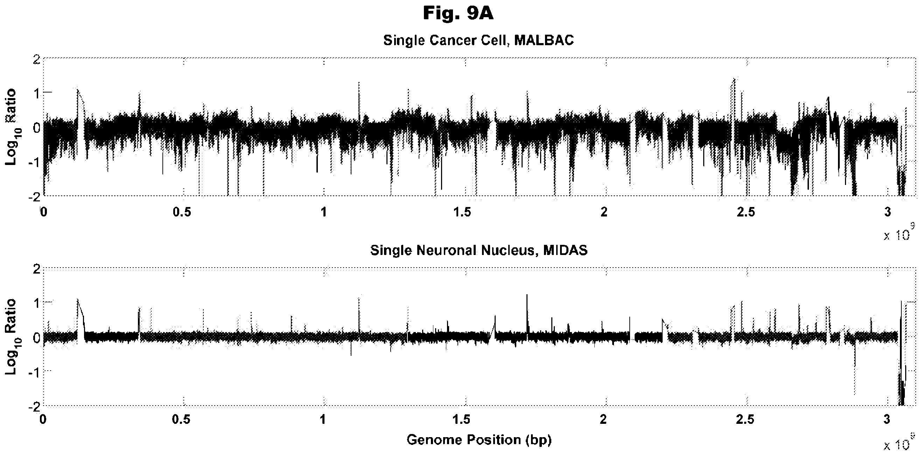

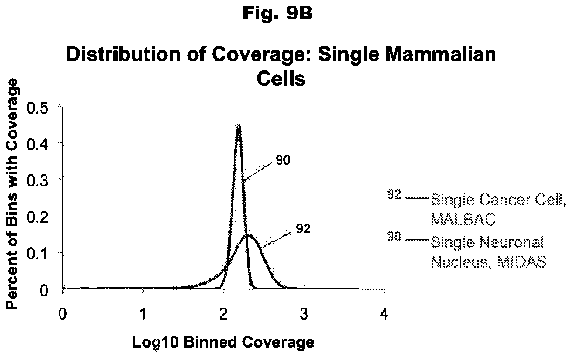

**Figure 9A-9B** is a series of graphs depicting a comparison of MIDAS amplification to MALBAC, a different method of amplifying nucleic acids. **Figure 9A** is a pair of graphs depicting MALBAC (top) and MIDAS (bottom), in which MIDAS and MALBAC show similar unbiased coverage across the genome. **Figure 9B** is a pair of graphs depicting MIDAS **90** displays a slightly better distribution of coverages when compared with MALBAC **92.**

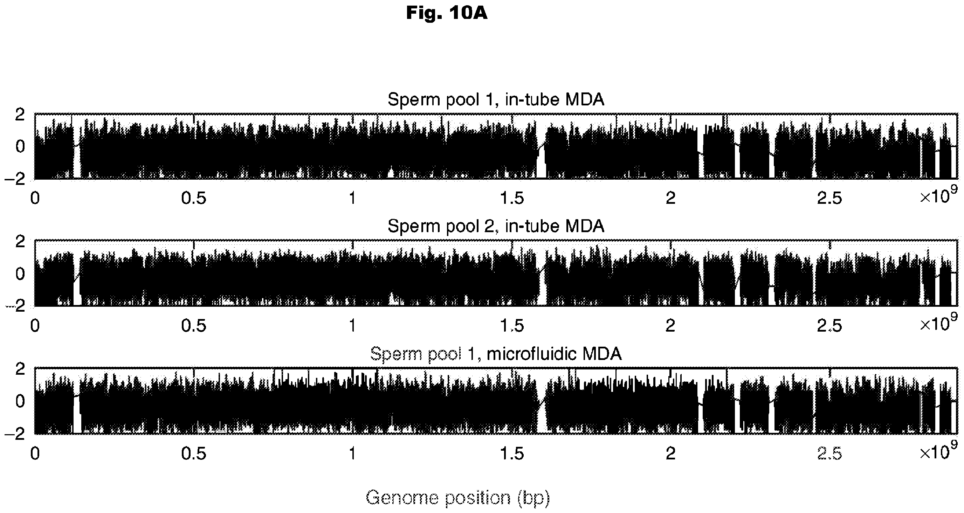

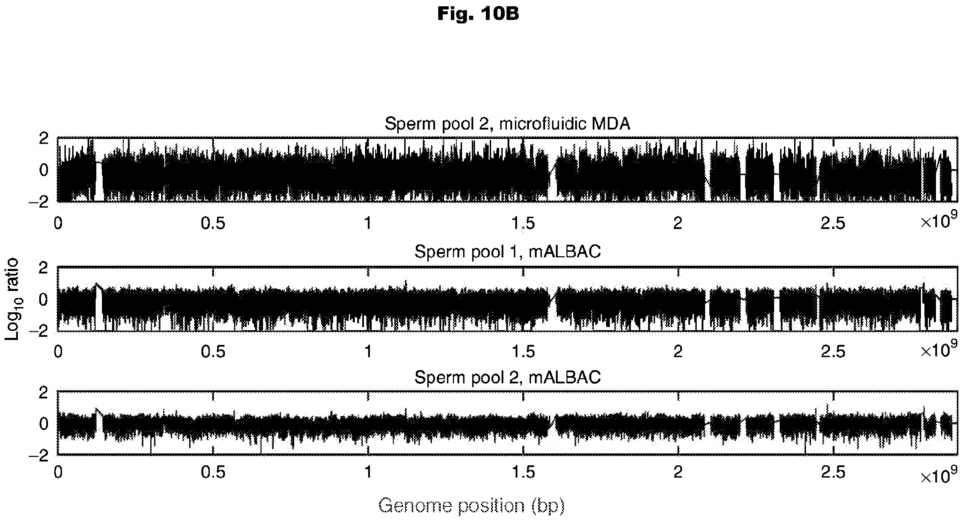

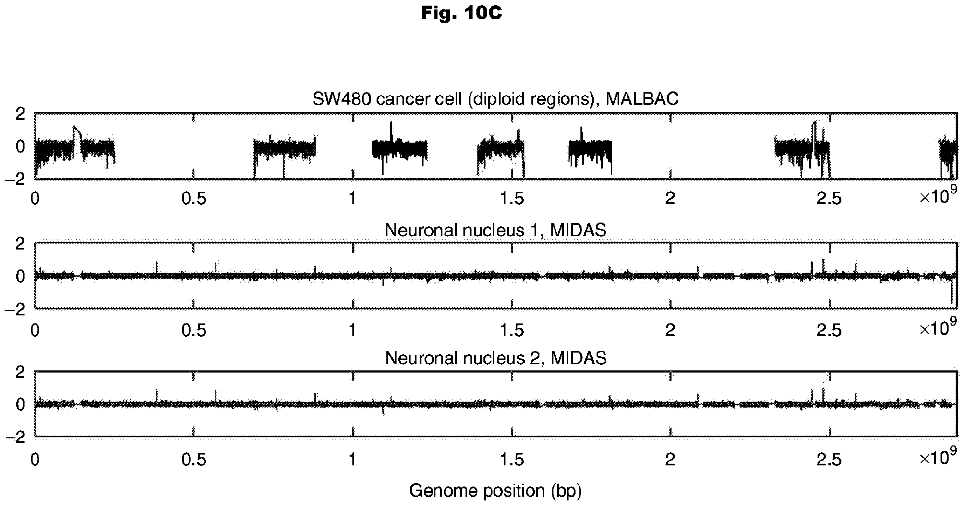

**Figures 10A-10C** are a series of graphs depicting a comparison of MIDAS amplification to previously published data for in-tube MDA⁴³, microfluidic MDA¹⁰ and MALBAC⁴⁴ for diploid regions of pools of two sperm cells and diploid regions of a single SW480 cancer cell processed using MALBAC³². Genomic positions were consolidated into variable bins of -60 kb in size previously determined to contain a similar read count ³⁰ and were plotted against the log10 ratio (y axis) of genomic coverage (normalized to the mean). For the cancer cell data, nondiploid regions have been masked out (white gaps between pink) to remove the bias generated by comparing a highly aneuploid cell to a primarily diploid cell. **Figure 10A** depicts results for sperm pool 1, in-tube MDA; sperm pool 2, in-tube MDA; and sperm pool 1, microfluidic MDA. **Figure 10B** depicts results for sperm pool 2, microfluidic MDA; sperm pool 1, mALBAC; and sperm pool 2, mALBAC. **Figure 10C** depicts results for SW480 cancer cell (diploid regions, MALBAC), Neuronal nucleus 1, MIDAS; and Neuronal nucleus 2, MIDAS.

### DETAILED DESCRIPTION

Amplification of sub-nanogram quantities of nucleic acids, for example the genome of a single cell, can be useful for a number of applications. Methods and manufactures for substantially unbiased amplification of nucleic acids are provided. A small quantity of nucleic acid, for example the genomic material of a single cell, may be amplified in a nanolitre-scale volume. The nanolitre-scale volume can provide for amplification in a high concentration of reactants. The amplification can comprise multiple strand displacement amplification (MDA). The amplification may be performed in a single reaction space, such as a well, thus minimizing moving parts. The amplification method can be readily scaled by simply increasing or decreasing a number of nanolitre-scale amplifications that are performed in parallel. A sequencing library may be prepared from the amplified nucleic acid. The library may comprise a random fragmentation and ligation library.

Genome sequencing of single cells can have a variety of applications including, but not limited to characterizing difficult-to-culture microorganisms and identifying somatic mutations in single cells from mammalian tissues. A major hurdle of this process can be bias in amplifying and making multiple copies of the genetic material from a single cell, a procedure known as polymerase cloning. Some examples herein provide a microwell displacement amplification system (MIDAS), a massively parallel polymerase cloning method in which single cells are randomly distributed into hundreds to thousands of microwells in nanolitre-scale volumes and simultaneously amplified for shotgun sequencing. In some examples, MIDAS dramatically reduces amplification bias by implementing polymerase cloning in nanolitre-scale reactions, allowing the *de novo* assembly of near-complete microbial genomes from single *E. coli* cells. In some examples, MIDAS allows detection of single-copy number changes in primary human adult neurons at 1-2 Mb resolution. MIDAS can facilitate the characterization of genomic diversity in many heterogeneous cell populations. It is further contemplated that as amplification reactions are performed in a single reaction environment, these reactions can be performed with minimal moving parts (for example, only a pippettor to add or remove solution from a reaction environment). Accordingly, amplification reactions can be performed with a high degree of reliability, while minimizing the need for additional components such as moving parts, and chasses and operating software for such moving parts. In some examples, amplification is performed in a single reaction environment. In some examples, the amplification is performed without the activity of fluidic channels or other fluidic system other than one or more pipettors for adding and/or removing solution from the reaction environment. In some examples, the amplification is performed in a reaction environment that is not in fluid communication with a network of fluidic channels, and is not configured for being in fluid communication with a network of fluidic channels.

Some examples allow for whole genome amplification of many single cells in parallel in an unbiased manner. Hundreds (or more) of cells can be amplified simultaneously in nanolitre volumes. Some examples include a low input sequencing library construction technique such that DNA directly from the whole genome amplification can be sequenced. The unbiased nature of amplification can allow for a myriad of downstream applications, including *de novo* assembly of unculturable bacteria and copy number variation calling of single mammalian cells.

According to some examples herein, methods of nucleic acid amplification are readily scalable. Depending on the desired number of amplification reactions to be performed, a number of nanolitre-scale reaction environments (for example wells) can be selected. Templates (e.g. single cells, or single cell genomes) can be diluted to a volume such that there is approximately no more than one template per reaction environment, and distributed among the desired number of reaction environments. In some examples, at least one substrate comprising a plurality of nanolitre-scale reaction environments is provided. If the desired number of reactions is less than the number of reaction environments on the substrate, only some of the reaction environments can be used. If the desired number of reactions is greater than the number of reaction environments on the substrate, two or more substrates can be used, for example 2, 3, 4, 5, 6, 7, 8, 9, 10, 11, 12, 13, 14, 15, 16, 17, 18, 19, 20, 30, 40, 50, 60, 70, 80, 90, or 100 substrates, including ranges between any two of the listed values. It is contemplated herein that scalability offers flexibility to an operator. Additionally, as amplification reactions can be performed with minimal moving parts, the number of amplification reactions can be readily scaled without any substantial customization or redesign of the substrate architecture (such as operating software, mechanical components, fluidic systems, and the like). Accordingly, a large number of amplification reactions can be performed in parallel. In some examples, at least 2 amplification reactions are performed in parallel, for example at least 2, 3, 4, 5, 2, 3, 4, 5, 6, 7, 8, 9, 10, 11, 12, 13, 14, 15, 16, 17, 18, 19, 20, 30, 40, 50, 60, 70, 80, 90, 100, 150, 200, 250, 300, 350, 400, 450, 500, 550, 600, 650, 700, 750, 800, 850, 900, 950, 1000,1500, 2000, 2500, 3000, 3500, 4000, 4500, 5000, 5500, 6000, 6500, 7000, 7500, 8000, 8500, 9000, 9500, or 10000 amplifications, including ranges between any two of the listed values.

### Nucleic acid amplification

Traditional whole genome amplification techniques for single cells can amplify genomes extremely biasedly. Small regions of the genome can be amplified greatly, whereas most of the genome can be amplified very little. Therefore, a large amount of sequencing effort can be required to resolve any of the genome. Downstream applications, such as *de novo* assembly or copy number variation calling, thus can be extremely difficult and inaccurate.

In some examples, whole genomes of single cells are amplified unbiasedly. In some examples, whole genomes of single cells are amplified substantially unbiasedly. As used herein "substantially unbiased" and pluralizations, conjugations, variations, and the like of this root term refers to amplification of a genome wherein, when the amplified genome is divided into at least 100 genomic bins that were previously determined such that each would contain a similar number of reads after mapping (*see, e.g.* ³⁰), the log₁₀ fold-amplification of at least 80% of the bins is within ±20% of the mean (i.e. for at least 80% percent of the genomic bins, the log₁₀ of the fold amplification, is no more than 20% more, and no less than 20% less than the mean number of copies genome-wide). In some examples, the log₁₀ fold-amplification of at least 80% of the bins is within ±20% of the mean, for example at least about 80%, 85%, 90%, 95%, 99%, or 99.9%. When whole genome amplification is substantially unbiased or unbiased, most of the genome can be amplified to a similar degree. Therefore, relatively little sequencing effort can be necessary for downstream analysis. *De novo* assembly can be accomplished and copy number variations can be called with a much greater accuracy.

As used herein, "nanolitre-scale" refers to a volume, for example in a reaction environment, of at least about one nanolitre and no more than about 50 nanolitres, more preferably about 5 nanolitres to about 30 nanolitres, more preferably about 10 nanolitres to about 25 nanolitres, for example about 12 nanolitres or about 20 nanolitres.

In some examples, cells are diluted and spread evenly across a loading area on a substrate, in which the loading area contains hundreds of nanolitre-scale reaction environments such that at least 99% of the reaction environments contain no more than 1 cell per well. In some examples, the substrate comprises a PDMS slide. After lysis and denaturing, the DNA can be amplified using multiple displacement amplification (MDA). The MDA reactants can be provided in buffer comprising polymerase, dNTP's, random oligonucleotides, and an amplification-detection moiety such as SYBR™ green dye. The MDA can be performed in a temperature controlled environment and in optical communication with a detector for amplification-detection moiety, such as a microscope. Without being limited by any theory, the small volume and consequent high concentration of template can allow for an unbiased amplification of the whole genome. Staining with an amplification-detection moiety, for example SYBR™ green, during amplification allows for positive amplifications to be observed due to an increase in detectable signal over time. Positive amplifications are then automatically or manually removed using a micromanipulator and deposited into tubes. Some examples include a low input sequencing library construction method capable of using sub nanogram inputs of DNA. The complex MDA amplicon can then be denatured and simple linear DNA created. The linear DNA can be used to construct a sequencing library. In some examples, transposons with Illumina sequencing adaptors (Nextera) then fragment the DNA while adding sequencing adapters. Accordingly, a sequencing library can be prepared. It is contemplated that nucleic acid amplified substantially unbiasedly can be used for a number of downstream applications, including any of a number of genome sequencing techniques known to the skilled artisan.

A variety of techniques for amplifying nucleic acid are known to the skilled artisan. Exemplary techniques for amplifying nucleic acid include, but are not limited to: polymerase chain reaction (PCR), strand displacement amplification (SDA), for example multiple displacement amplification (MDA), loop-mediated isothermal amplification (LAMP), ligase chain reaction (LCR), immuno-amplification, and a variety of transcription-based amplification procedures, including transcription-mediated amplification (TMA), nucleic acid sequence based amplification (NASBA), self-sustained sequence replication (3SR), and rolling circle amplification. See, e.g., Mullis, "Process for Amplifying, Detecting, and/or Cloning Nucleic Acid Sequences," U.S. Pat. No. 4,683,195; Walker, "Strand Displacement Amplification," U.S. Pat. No. 5,455,166; Dean et al, "Multiple displacement amplification," U.S. Pat. No. 6,977,148; Notomi et al., "Process for Synthesizing Nucleic Acid," U.S. Pat. No. 6,410,278; Landegren et al. U.S. Pat. No. 4,988,617 "Method of detecting a nucleotide change in nucleic acids"; Birkenmeyer, "Amplification of Target Nucleic Acids Using Gap Filling Ligase Chain Reaction," U.S. Pat. No. 5,427,930; Cashman, "Blocked-Polymerase Polynucleotide Immunoassay Method and Kit," U.S. Pat. No. 5,849,478; Kacian et al., "Nucleic Acid Sequence Amplification Methods," U.S. Pat. No. 5,399,491; Malek et al., "Enhanced Nucleic Acid Amplification Process," U.S. Pat. No. 5,130,238; Lizardi et al., BioTechnology, 6:1197 (1988); Lizardi et al., U.S. Pat. No. 5,854,033 "Rolling circle replication reporter systems,". Preferably, MDA can be used in accordance with some examples herein. MDA can comprise annealing random oligonucleotide primers to a template nucleic acid, and extending the oligonucleotide primers forward to the annealing site of the most immediate downstream oligonucleotide primer so as to form branched amplified nucleic acid. MDA can be performed at a constant temperature, and compared to conventional PCR can produce relatively large products with a relatively low error rate. A variety of MDA reagents can be used in accordance with examples herein. In some examples, MDA is performed with a strand-displacement polymerase In some examples, the strand displacement polymerase comprises a high-fidelity DNA polymerase. for example Φ29 DNA polymerase.

The fold amount of amplification that occurs can depend on the amount of template, and the total mass of reactants. According to some examples herein, amplification is performed until saturation (e.g. until additional cycles of amplification are no longer in a logarithmic phase, so that the additional cycles produce few to no additional amplicons). Without being limited by any theory, it is contemplated that the total amount of amplification is proportional to the total mass of the reaction, and inversely proportional to the size of the template being amplification. Accordingly, by way of example, given the same reaction mass and amplification until saturation a 1Mb genome would be amplified approximately 10-fold more than a 10Mb genome.

Without being limited by any theory, it is contemplated herein that a high concentration of amplification reactants and template can facilitate substantially unbiased amplification of all or substantially all of the template, for example genomic material. So as to provide a high concentration of reactants, including, but not limited to, template, the ratio of template to reaction volume can be relatively high. Accordingly, in some examples, the nanolitre-scale reaction environments are configured for a high ratio of genomic material to reaction volume. In some examples, the nanolitre-scale reaction environments are configured for at least about 0.02 megabases of genomic material per nanolitre of reaction volume, for example at least about 0.02, 0.03, 0.05, 0.1, 0.15, 0.2, 0.25, 0.3, 0.35, 0.4., 0.45, 0.5, 0.6, 0.7, 0.8, 0.9, 1, 2, 3,4 , 5, 6, 7, 8, 9, 10, 20, 30, 40, 50, 60, 70, 80, 90, 100, 150, 200, 250, 300, 350, 400, 450, 500, 550, 600, 650, 700, 750, 800, 850, 900, 950, 1000, 1500, 2000, 2500, 3000, 4500, or 5000 megabases of genomic material per nanolitre, including ranges between any two of the listed values. In some examples, the nanolitre-scale reaction environments are configured for at least about 0.03 megabases of genomic material per nanolitre of reaction. In some examples, the nanolitre-scale reaction environments are configured for at least about 0.3 megabases of genomic material per nanolitre of reaction. In some examples, the nanolitre-scale reaction environments are configured for at least about 100 megabases of genomic material per nanolitre of reaction. In some examples, the nanolitre-scale reaction environments are configured for at least about 200 megabases of genomic material per nanolitre of reaction. It is further contemplated herein that the nanolitre-scale reaction environments can be configured so that substantially each nanolitre-scale reaction environment comprises only one genome (or cell comprising a genome) when a liquid comprising diluted whole cells or fractions thereof is applied to a substrate as described herein. Accordingly, in some examples, each nanolitre-scale reaction environment is configured so that at least about 95% of the nanolitre-scale reaction environments comprises only one cell after administration of the solution comprising cells or fragments thereof, for example at least about 95%, 96%, 97%, 98%, 99%, 99.5%, 99.6%, 99.7%, 99.8%, 99.9%, or 99.99%.

While substantially unbiased amplification can be useful for many applications, one useful application includes genome sequencing. It is contemplated that the substantially unbiased amplification amplification of all or substantially all of the template genome at a coverage level that is useful for sequencing. In some examples, the nanolitre-scale reaction environments are configured for amplifying at least about 90% of the entire genome therein with >1x coverage, for example at least about 90%, 91%, 92%, 93%, 94%, 95%, 96%, 97%, 98%, 99%, 99.5%, or 99.9%, including ranges between any two of the listed values.

In some examples, unbranched amplicons are produced for use in library construction. As used herein, "substantially all amplicons are unbranched" and the like refers to at least about 70% of the amplicons (for example, about 70%, 75%, 80%, 85%, 90%, 95%, 97%, 98%, 99%, or 99.9%) do not have a branch characteristic of multiple strand displacement, but rather, are unbranched double-stranded DNA molecules. Without being limited by any theory, it is noted that MDA products are typically highly branched. In some examples, unbranched amplicons can be produced from MDA products by contacting the MDA products with DNA polymerase I.

A variety of sequencing techniques are known to the skilled artisan, and can be used in accordance with examples herein. The selection of a sequencing technique can depend on a variety of factors, for example the size and characteristics of a genome being amplified. As a number of examples herein comprise or are compatible with massively parallel amplification and sequencing, sequencing techniques compatible with rapid, large-scale "next-generation" sequencing can be useful in accordance with some examples herein. Exemplary sequencing techniques include Illumina™ (Solexa) sequencing (Illumina), Ion Torrent™ sequencing (Life Technologies), SOLiD™ sequencing (Life Technologies), and the like.

### Amplification-detection moieties

In some examples, an amplification-detection moiety is used to monitor the progress of amplification. As used herein, "amplification-detection moiety" refers broadly to any of number of detectable moieties that produce a detectable type or intensity of signal in the presence of amplification product, for example double-stranded nucleic acid, but do not produce the signal (or produce only low-level or background signal) in the absence of amplification product. A first class of amplification-detection moieties includes dyes that bind specifically to double-stranded DNA, for example intercalating agents. These dyes have a relatively low fluorescence when unbound, and a relatively high fluorescence upon binding to double-stranded nucleic acids. As such, dyes that selectively detect double-stranded can be used to monitor the accumulation of double strained nucleic acids during an amplification reaction. Examples of dyes that selectively detect double-stranded DNA include, but are not limited to SYBR™ Green I dye (Molecular Probes), SYBR™ Green II dye (Molecular Probes), SYBR™ Gold dye (Molecular Probes), Picogreen,dye (Molecular Probes), Hoechst 33258 (Hoechst AG), and cyanine dimer families of dyes such as the YOYO family of dyes (e.g. YOYO-1 and YOYO-3), the TOTO family of dyes (e.g. TOTO-1 and TOTO-3), and the like. Other types of amplification-detection moieties employ derivatives of sequence-specific nucleic acid probes. For example, oligonucleotide probes labeled with one or more dyes, such that upon hybridization to a template nucleic acid, a detectable change in fluorescence is generated. Exemplary amplification-detection moieties in this class include, but are not limited to Taqman™ probes, molecular beacons, and the like. While non-specific dyes may be desirable for some applications, sequence-specific probes can provide more accurate measurements of amplification. One configuration of sequence-specific probe can include one end of the probe tethered to a fluorophore, and the other end of the probe tethered to a quencher. When the probe is unhybridized, it can maintain a stem-loop configuration, in which the fluorophore is quenched by the quencher, thus preventing the fluorophore from fluorescing. When the probe is hybridized to a template nucleic sequence, it is linearized, distancing the fluorophore from the quencher, and thus permitting the fluorophore to fluoresce. Another configuration of sequence-specific probe can include a first probe tethered to a first fluorophore of a FRET pair, and a second probe tethered to a second fluorophore of a FRET pair. The first probe and second probe can be configured to hybridize to sequences of an amplicon that are within sufficient proximity to permit energy transfer by FRET when the first probe and second probe are hybridized to the same amplicon.

In some examples, an amplification-detection moiety is used to quantify the double-stranded DNA in each reaction environment. Accordingly, in some examples, the products of reaction environments in which a desired amount of amplification has occurred can be selected for downstream applications such as construction of sequencing libraries. Thus, methods according to some examples herein can minimize the use of reagents and other resources by only constructing sequencing libraries for single-cell genomes that were actually amplified, and for reducing a need for preparing redundant libraries as a "back-up" against reaction environments that did not amplify.

In some examples, the sequence-specific probe comprises an oligonucleotide that is complementary to a sequence to be amplified, and is conjugated to a fluorophore. In some examples, the probe is conjugated to two or more fluorophores. Examples of fluorophores include: xanthene dyes, e.g., fluorescein and rhodamine dyes, such as fluorescein isothiocyanate (FITC), 2-[ethylamino)-3-(ethylimino)-2-7-dimethyl-3H-xanthen-9-yl]benzoic acid ethyl ester monohydrochloride (R6G)(emits a response radiation in the wavelength that ranges from about 500 to 560 nm), 1,1,3,3,3',3'-Hexamethylindodicarbocyanine iodide (HIDC) (emits a response radiation in the wavelength that ranged from about 600 to 660 nm), 6-carboxyfluorescein (commonly known by the abbreviations FAM and F), 6-carboxy-2',4',7',4,7-hexachlorofluorescein (HEX), 6-carboxy-4',5'-dichloro-2',7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, e.g. Cy3, Cy5 and Cy7 dyes; coumarins, e.g., umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3 (emits a response radiation in the wavelength that ranges from about 540 to 580 nm), Cy5 (emits a response radiation in the wavelength that ranges from about 640 to 680 nm), etc; BODIPY dyes and quinoline dyes. Specific fluorophores of interest include: Pyrene, Coumarin, Diethylaminocoumarin, FAM, Fluorescein Chlorotriazinyl, Fluorescein, R110, Eosin, JOE, R6G, HIDC, Tetramethylrhodamine, TAMRA, Lissamine, ROX, Napthofluorescein, Texas Red, Napthofluorescein, Cy3, and Cy5, and the like.

In some examples, the sequence-specific probe is conjugated to a quencher. A quencher can absorb electromagnetic radiation and dissipate it as heat, thus remaining dark. Example quenchers include Dabcyl, NFQ's, such as BHQ-1 or BHQ-2 (Biosearch), IOWA BLACK FQ (IDT), and IOWA BLACK RQ (IDT). In some examples, the quencher is selected to pair with a fluorophore so as to absorb electromagnetic radiation emitted by the fluorophore. Flourophore/quencher pairs useful in the compositions and methods disclosed herein are well-known in the art, and can be found, e.g., described in S. Marras, "Selection of Fluorophore and Quencher Pairs for Fluorescent Nucleic Acid Hybridization Probes" available at the world wide web site molecular-beacons.org/download/marras,mmb06%28335%293.pdf.

In some examples, a fluorophore is attached to a first end of the sequence-specific probe, and a quencher is attached to a second end of the probe. Attachment can include covalent bonding, and can optionally include at least one linker molecule positioned between the probe and the fluorophore or quencher. In some examples, a fluorophore is attached to a 5' end of a probe, and a quencher is attached to a 3' end of a probe. In some examples, a fluorophore is attached to a 3' end of a probe, and a quencher is attached to a 5' end of a probe. Examples of probes that can be used in quantitative nucleic acid amplification include molecular beacons, SCORPIONS™ probes (Sigma) and TAQMAN™ probes (Life Technologies).

### Substrates

Substrates comprising a plurality of nanolitre-scale reaction environments can be used in accordance with some examples herein.

In some examples, the substrate comprises several loading areas, and a plurality of nanolitre-scale reaction environments in fluid communication with each loading area. In some examples, applying to a loading area a solution having the total volume of the nanolitre-scale reaction environments for that loading area, and single genomes (for example single cells, or isolated genomes of single cells) at a dilution of about 0.1 genome per reaction environment can result in 99% of the reaction environments in that loading area comprising no more than a single genome (or single cell comprising that genome). For example, if each loading area of the substrate comprises 255 microwell reaction environments, each having a diameter of about 400µm and a depth of about 100µm (for a volume of about 12 nl), applying 3µl of a solution comprising 0.1 cells per microwell (e.g. 26 cells), about 99.5% of the microwells will comprise no more than one cell. It is noted that this number was confirmed via SEM microscopy (*see* **Fig. 1B**).

An exemplary substrate **10** in accordance with some examples herein is schematically illustrated in **Fig. 1A****.** The substrate can comprise several loading areas **12,** which are not in fluid communication with each other. In some examples, the substrate comprises at least 3 loading areas, for example, 3, 4, 5, 6, 7, 8, 9, 10, 11, 12, 13, 14, 15, 16, 17, 18, 19, 20, 21, 22, 23, 24, 25, 30, 35, 40, 50, 55, 60, 65, 70, 75, 80, 85, 90, 95, 100, 150, 200, 250, 300, 350, 400, 450, or 500 loading areas, including ranges between any two of the listed values. In some examples, each loading area is configured to be loaded directly by a pipette without any intervening fluidic channels (e.g. microfluidic or nanofluidic channels). The pipette can be manually operated or automatically operated. Each loading area **12** can comprise, or can be in fluid communication with a plurality of nanolitre-scale reaction environments **14,** for example microwells. The number of nanolitre-scale reaction environments can be useful for increasing the likelihood that no each reaction environment comprises no more than one genome (or single cell comprising a genome). In some examples, each loading area **12** comprises at least about 100 nanolitre-scale reaction environments, for example about 100, 150, 200, 250, 300, 350, 400, 450, 500, 550, 600, 650, 700, 750, 800, 850, 900, 950, 1000, 1100, 1200, 1300, 1400, 1500, 1600, 1700, 1800, 1900, 2000, 3000, 4000, or 5000 nanolitre-scale reaction environments, including ranges between any two of the listed values. In some examples, each nanolitre-scale reaction environment **14** has a volume of no more than 30 nanolitres, for example about 30, 29, 28, 27, 26, 25, 24, 23, 22, 21, 20, 19, 18, 17, 16, 15, 14, 13, 12, 11, 10, 9, 8, 7, 6, 5, 4, 3, 2, or 1 nanolitres, including ranges between any two of the listed values. In some examples, each nanolitre-scale reaction environment **14** has a volume of no more than 20 nanolitres. In some examples, each nanolitre-scale reaction environment **14** has a volume of no more than 12 nanolitres. In some examples, each nanolitre-scale reaction environment **14** has a volume of about 20 nl. In some examples, each nanolitre-scale reaction environment **14** has a volume of about 12 nl. In some examples, each nanolitre-scale reaction environment has a diameter-to-depth ratio of about 4:1, for example about 2:1, 3:1, 4:1, 5:1, 6:1, 7:1, or 8:1. For example, a round nanolitre-scale reaction environment having a diameter of about 400µm and a depth of about 100µm would have a volume of about 12nl.

It is recognized that each loading area can be loaded with a separate sample, so that multiple samples can be amplified on the same substrate in parallel (one sample in each loading area). Accordingly, in some examples, the number of samples being amplified in parallel can readily be scaled up or down. For example, if the number of samples is less than or equal to the total number of loading areas on the substrate, the appropriate number of loading areas can be selected for parallel reactions. If the number of samples is greater than the total number of loading areas on the substrate, two or more substrates can be used to accommodate the total number of samples.

In some examples, the substrate **100** comprises 16 loading areas **12,** and each loading area **12** comprises 255 nanolitre-scale loading environments **14.** Each nanolitre-scale reaction environment **14** can have a diameter of about 400µm and a depth of about 100µm, for a volume of about 12nl. The substrate can comprise PDMS. Each loading area can have a height of about 7mm and a width of about 7mm. The loading areas can be arranged in a pattern on the substrate.

In some examples, the substrate further includes a detector for amplification-detection moieties. The detector need not be attached to the substrate. For example, the substrate can be positioned in optical communication with a fluorescent microscope, and optionally a camera. In accordance with some examples herein, an amplification-detection moiety can be present in the nanolitre-scale reaction environments, and can indicate when a desired amount of amplification of nucleic acid has occurred in a particular nanolitre-scale reaction environment. Accordingly, in some examples, the detector is configured to detect nanolitre-scale reaction environments in which a desired amount of amplification has occurred. In some examples, a manual user can select one or more nanolitre-scale reaction environments for downstream applications such as library construction based on the signal detected by the detector. In some examples, one or more nanolitre-scale reaction environments are automatically selected for downstream applications such as for library construction based on the amount of signal detected by the detector.

In some examples, the substrate further comprises a pipettor for withdrawing amplified nucleic acid from a selected nanolitre-scale reaction environment. The pipettor can be configured to withdraw nanolitre-scale volumes or less from the selected well. In some examples, the pipettor comprises a pipette having a diameter less than the diameter of the nanolitre-scale reaction environment. In some examples, the pipette has a diameter of no more than about 50µm, for example about 50µm, 45, 40, 35, 30, 25, 20, 15, 10, or 5µm, including ranges between any two of the listed values. In some examples, the pipette has a diameter of about 30µm. In some examples, the pipette is a glass pipette. The pipette can be sterile. In some examples, the pipettor is under the mechanical control of a manual micromanipulator so that a user can manually select a nanolitre-scale reaction environment of interest for withdrawing liquid, for example amplified nucleic acid. In some examples, the pipettor is under the mechanical control of an automatic micromanipulator in data communication with a detector as described herein, so that the pipettor can automatically withdraw liquid from a nanolitre-scale reaction environment exhibiting a desired level of amplification.

In some examples, the genome of microbial and/or human cells is sequenced. Some examples include assembly of genomes of single bacterial cells with very little sequencing effort. Some examples include calling copy number variations on single human neurons down to a 1-2 megabase resolution.

Methods and manufactures in accordance with some examples herein can be useful for one or more of: *De novo* assembly of unculturable bacteria in the human gut; De novo assembly of unculturable bacteria in heterogeneous environments such as sea water; Copy number variation calling on single neurons; Copy number variation calling on single cancerous cells or circulating tumor cells; and haplotyping, for example Human haplotyping.

In some examples, the genome of a single cell is amplified. In some examples the cell is a human cell. In some examples, the cell is a microbial cell. In some examples, the cell is a bacterial cell. In some examples, the cell is from a substantially unculturable strain. As used herein, "substantially unculturable" and variations thereof refer to a strain that, when cultured under normal laboratory conditions, fewer than 20% of replicates of that strain will reach a logarithmic growth phase, for example fewer than 20%, 15%, 10%, 5%, 2%, 1%, or 0.1%.

For previous techniques, a major technical challenge was the highly uneven amplification of the one or two copies of each chromosome in a single cell. This high amplification bias leads to difficulties in assembling microbial genomes *de novo* and inaccurate identification of copy number variants (CNV) or heterozygous single nucleotide changes in single mammalian cells. Recent developments of bias-tolerant algorithms^{11, 12} have greatly mitigated the effects of uneven read depth on *de novo* genome assembly and CNV calling, yet an unusually high sequencing depth is still required, making this approach impractical for organisms with large genome sizes.

Several strategies have been previously developed to reduce amplification bias, including reduced reaction volume^{13, 14} and supplementing amplification reactions with single-strand binding proteins or Threhalose^{15, 16}. Post-amplification normalization by digesting highly abundant sequences with a duplex-specific nuclease has also been utilized to markedly reduce bias¹⁷. Despite these efforts, amplification bias still remains the primary technical challenge in single-cell genome sequencing. Using cells that contain multiple copies of the genome or multiple clonal cells has been the only viable solution to achieve near complete genome coverage with MDA^{18, 19}. Without being bound by any particular theory, we reasoned that amplification is always bias-prone, and that by limiting the amplification magnitude to "just-enough" for sequencing in accordance with some examples herein, we could potentially reduce the bias. In addition, we supposed that reducing the reaction volume by -1000 fold to nanolitre level and thus dramatically increasing the effective concentration of the template genome might reduce contamination and improve primer annealing and hence amplification uniformity^{13, 14}. To these ends, we developed the microwell displacement amplification system (MIDAS) in accordance with some examples herein, a microwell-based platform that allows for highly parallel polymerase cloning of single cells in thousands of nanolitre reactors of 12 nL in volume, the smallest volume that has been implemented to date to the best knowledge of Applicants. Coupled with a low-input library construction method, we achieved highly uniform coverage in the genomes of both microbial and mammalian cells. We demonstrated substantial improvement both in *de novo* genome assembly from single microbial cells and in the ability to detect small somatic copy number variants in individual human adult neurons with minimal sequencing effort.

Due to the extreme bias that can be caused by whole genome amplification from a single DNA molecule, genomic analysis of single cells has traditionally been a challenging task. Traditionally, a large amount of sequencing resources can be required to produce a draft quality genome assembly or determine a low-resolution copy number variation profile due to amplification bias and coverage dropout. MIDAS addresses this issue through the use of nanolitre scale volumes to generate nanogram level amplicons and the use of a low-input transposon-based library construction method. Compared to the traditional single-cell library construction and sequencing protocol, MIDAS provides a more uniform, higher-coverage, and lower cost way to analyze single cells from a heterogeneous population.

MIDAS was applied to single *E. coli* cells and resolved nearly the entire genome with relatively low sequencing depth. Additionally, using *de novo* assembly on MIDAS libraries, over 90 percent of the genome was assembled. Thus, in some examples, MIDAS is applied to an uncultivated organism to provide a draft quality assembly with more genes covered and less sequencing resource expenditure. Currently, a majority of unculturable bacteria are analyzed metagenomically as part of a mixed population rather than individually. Although metagenomics allows for the discovery of novel genes, individual sequences cannot be resolved. The biased nature of traditional MDA-based methods when applied to single cells has proved single cell microbial analysis challenging in terms of *de novo* genome assembly. Despite recent success in analyzing partially assembled single cell genomes⁷, the full potential of single cell genomics remains to be fully explored. As such, in some examples the use of MIDAS on heterogeneous environmental samples, novel single-cell organisms and genes can be easily discovered and characterized in a low-cost and high-throughput manner, allowing a much higher-resolution and more complete analysis of single bacterial cells.

In some examples, MIDAS is applied to the analysis of copy number variation in single human neuronal nuclei. With a low amount of sequencing effort, MIDAS was able to systematically call single copy number changes of 2 million base pairs or larger in size. It has been shown recently that, in human adult brains, post-mitotic neurons in different brain regions exhibited various levels of DNA content variation (DCV)²⁹. The exact genomic regions that associate with DCV have been difficult to map to single neurons because of the amplification bias with existing MDA-based methods. CNVs in single tumor cells have been successfully characterized with a PCR-based whole genome amplification method⁸. However, tumor cells tend to be highly aneuploid and exhibit copy number changes of larger magnitude, which are more easily detected. The applicability of this strategy to other primary cell types with more subtle CNV events remains unclear. We have demonstrated that MIDAS greatly reduces the variability of single cell analysis to a level such that a small single-copy change is detectible, allowing characterization of much more subtle copy number variation. MIDAS can be used to simultaneously probe into the individual genomes of many cells from patients with neurological diseases, and thus will allow identification of a range of structural genomic variants and eventually allow accurate determination of the influence of somatic CNVs on brain disorders in a high-throughput manner.

In some examples, MIDAS compares very favorably to traditional MDA-based methods. Recently, another single cell sequencing method that dramatically reduces amplification bias and increases genomic coverage was reported. Known as MALBAC, this method incorporates a novel enzymatic strategy to amplify single DNA molecules initially through quasi-linear amplification to a limited magnitude prior to exponential amplification and library construction³². MALBAC was implemented in microliter reactions in conventional reaction tubes. In contrast, MIDAS represents an orthogonal strategy by adapting MDA to a microwell platform. It will therefore be more easily able to analyze a larger number of single cells in parallel in a single experiment. While both MIDAS and MALBAC show relatively unbiased amplification across the genome (**Figs. 9A-9B**), MIDAS shows less variability in coverage distribution, making it more suitable for CNV calling with less sequencing effort. Additionally, unlike MIDAS, MALBAC has not been demonstrated on femtogram level DNA inputs, which is required for genome sequencing of single microbial cells. Finally, the error rate of MALBAC is roughly 100-fold higher than MDA due to the difference in DNA polymerases used.

MIDAS can provide researchers with a powerful tool for many other applications, including high-coverage end-to-end haplotyping of mammalian genomes or probing *de novo* CNV events at the single-cell level during the induction of pluripotency or stem cell differentiation³³. MIDAS can allow for efficient high-throughput sequencing of a variety of organisms at a relatively low price. This new technology should help propel single cell genomics, enhance our ability to identify diversity in multicellular organisms, and lead to the discovery of thousands of new organisms in various environments.

### EXAMPLES

### Methods

With reference to Examples 1-5, the following methods were used. The skilled artisan will appreciate that the following methods can readily be used or adapted or modified in accordance with some examples herein:

### Microwell Array Fabrication

Microwell arrays were fabricated from polydimethylsiloxane (PDMS). Each array was 7 mm x 7 mm, with 2 rows of 8 arrays per slide and 156 microwells per array. The individual microwells were 400 µm in diameter and 100 um deep (-12 nL volume), and were arranged in honeycomb patterns in order to minimize space in between the wells. To fabricate the arrays, first, an SU-8 mold was created using soft lithography at the Nano3 facility at UC San Diego. Next, a 10:1 ratio of polymer to curing agent mixture of PDMS was poured over the mold. Finally, the PDMS was degassed and cured for 3 hours at 65 C.

### Bacteria and Neuron Preparation

*E. coli* K12 MG1655 was cultured overnight, collected in log-phase, and washed 3x in PBS. After quantification, the solution was diluted to 10 cells/µL. Human neuronal nuclei were isolated as previously described^{29, 34} and fixed in ice-cold 70% ethanol. Nuclei were labeled with a monoclonal mouse antibody against NeuN (1:100 dilution) (Chemicon, Temecula, CA) and an AlexaFluor 488 goat anti-mouse IgG secondary antibody (1:500 dilution) (Life Technologies, San Diego, CA). Nuclei were counterstained with propidium iodide (50ug/ml) (Sigma, St. Louis, MO) in PBS solution containing 50 ug/ml RNase A (Sigma) and chick erythrocyte nuclei (Biosure, Grass Valley, CA). Nuclei in the G1/G0 cell cycle peak, determined by propidium iodide fluorescence, were electronically gated on a Becton Dickinson FACS-Aria II (BD Biosciences, San Jose, CA) and selectively collected based on NeuN+ immunoreactivity.

### Cell Seeding, Lysis, and Multiple Displacement Amplification

All reagents not containing DNA or enzymes were first exposed to ultraviolet light for 10 minutes prior to use. The PDMS slides were treated with oxygen plasma to make them hydrophilic and ensure random cell seeding. The slides were then treated with 1% bovine serum albumin (BSA) (EMD Chemicals, Billerica, MA) in phosphate buffered saline (PBS) (Gibco, Grand Island, NY) for 30 minutes and washed 3x with PBS to prevent DNA from sticking to the PDMS. The slides were completely dried in a vacuum prior to cell seeding. Cells were diluted to a concentration of 10 cells/µL, and 3 µL of cell dilution was added to each array (30 cells total per array).

Initially, to verify that cell seeding adhered to the Poisson distribution, cells were stained with 1x SYBR green and viewed under a fluorescent microscope. Proper cell distribution was further confirmed with SEM imaging. For SEM imaging, chromium was sputtered onto the seeded cells for 6 seconds to increase conductivity. Note that the imaging of cell seeding was only used to confirm the theoretical Poisson distribution and not performed during actual amplification and sequencing experiments due to the potential introduction of contamination.

After seeding, cells were left to settle into the wells for 10 minutes. The seeded cells were then lysed either with 300 U ReadyLyse lysozyme at 100 U/µL (Epicentre, Madison, WI) and incubation at room temperature for 10 minutes, or with 5 1 minute freeze/thaw cycles using a dry ice brick and room temperature in a laminar flow hood. After lysis, 4.5 µL of alkaline lysis (ALS) buffer (400 mM KOH, 100 mM DTT, 10 mM EDTA) was added to each array and incubated on ice for 10 minutes. Then, 4.5 µL of neutralizing (NS) buffer (666 mM Tris-HCl, 250 mM HCL) was added to each array. 11.2 µL of MDA master mix (1x buffer, 0.2x SYBR green I, 1 mM dNTP's, 50 µM thiolated random hexamer primer, 8U phi29 polymerase, Epicentre, Madison, WI) was added and the arrays were then covered with mineral oil. The slides were then transferred to the microscope stage enclosed in a custom temperature and humidity controlled incubator set to 30 C. Images were taken at 30-minute intervals for 10 hours using a 488 nm filter.

### Image Analysis

Images were analyzed with a custom Matlab script to subtract background fluorescence. Because SYBR Green was added to the MDA master mix, fluorescence under a 488 nm filter was expected to increase over time for positive amplifications. If a digital profile of fluorescent wells with increasing fluorescence over time was observed (approximately 10-20 wells per array), the array was kept. If no wells fluoresced, amplification failed and further experiments were stopped. Alternatively, if a majority of the wells fluoresced, the array was considered to be contaminated and subsequent analysis was similarly stopped. If 2 abutting wells fluoresced, neither was extracted due to the higher likelihood of more than one cell in each well existing (as in this case, seeding was potentially non-uniform).

### Amplicon Extraction

1 mm outer diameter glass pipettes (Sutter, Novato, CA) were pulled to -30 um diameters, bent to a 45 degree angle under heat, coated with SigmaCote (Sigma, St. Louis, MO), and washed 3 times with dH₂0.

Wells with positive amplification were identified using the custom Matlab script described above. A digital micromanipulation system (Sutter, Novato, CA) was used for amplicon extraction. The glass pipette was loaded into the micromanipulator and moved over the well of interest. The microscope filter was switched to bright field and the pipette was lowered into the well. Negative pressure was slowly applied, and the well contents were visualized proceeding into the pipette. The filter was then switched back to 488 nm to ensure the well was no longer fluorescent. Amplicons were deposited in 1 µL dH₂0.

### Amplicon Quantification

For quantification of microwell amplification, 0.5 µL of amplicon was amplified a second time using MDA in a 20 µL PCR tube reaction (1x buffer, 0.2x SYBR green I, 1 mM dNTP's, 50 mM thiolated random hexamer primer, 8U phi29 polymerase). After purification using Ampure XP beads (Beckman Coulter, Brea, CA), the 2^{nd} round amplicon was quantified using a Nanodrop spectrophotometer. The 2^{nd} round amplicon was then diluted to 1 ng, 100 pg, 10 pg, 1 pg, and 100 fg to create an amplicon ladder. Subsequently, the remaining 0.5 µL of the 1^{st} round amplicon was amplified using MDA along with the amplicon ladder in a quantitative PCR machine. The samples were allowed to amplify to completion, and the time required for each to reach 0.5x of the maximum fluorescence was extracted. The original amplicon concentration could then be interpolated.

### Low-input library construction

1.5 µL of ALS buffer was added to the extracted amplicons to denature the DNA followed by a 3-minute incubation at room temperature. 1.5 µL of NS buffer was added on ice to neutralize the solution. 10 U of DNA Polymerase I (Invitrogen, Carlsbad, CA) was added to the denatured amplicons along with 250 nanograms of unmodified random hexamer primer, 1 mM dNTPs, 1x Ampligase buffer (Epicentre, Madison, Wi), and 1x NEB buffer 2 (NEB, Cambridge, MA). The solution was incubated at 37 C for 1 hour, allowing second strand synthesis. 1 U of Ampligase was added to seal nicks and the reaction was incubated first at 37 C for 10 minutes and then at 65 C for 10 minutes. The reaction was cleaned using standard ethanol precipitation and eluted in 4 µL water.

Nextera transposase enzymes (Epicentre, Madison, WI) were diluted 100 fold in 1x TE buffer and glycerol. 10 µL transposase reactions were then conducted on the eluted amplicons after addition of 1 µL of the diluted enzymes and 1x tagment DNA buffer. The reactions were incubated for 5 minutes at 55 C for mammalian cells and 1 minute at 55 C for bacterial cells. 0.05 U of protease (Qiagen, Hilden, Germany) was added to each sample to inactivate the transposase enzymes; the protease reactions were incubated at 50 C for 10 minutes followed by 65 C for 20 minutes. 5 U Exo minus Klenow (Epicentre, Madison, WI) and 1 mM dNTP's were added and incubated at 37 C for 15 minutes followed by 65 C for 20 minutes. Two stage quantitative PCR using 1x KAPA Robust 2G master mix (Kapa Biosystems, Woburn, MA), 10 µM Adapter 1, 10 µM barcoded Adapter 2 in the first stage, and 1x KAPA Robust 2G master mix, 10 µM Illumina primer 1, 10 µM Illumina primer 2, and 0.4x SYBR Green I in the second stage was performed and the reaction was stopped before amplification curves reached their plateaus. The reactions were then cleaned up using Ampure XP beads in a 1:1 ratio. A 6% PAGE gel verified successful tagmentation reactions.

### Mapping and De novo Assembly of Bacterial Genomes

Bacterial libraries were size selected into the 300-600 bp range and sequenced in an Illumina Genome Analyzer IIx, Illumina HiSeq, or Illumina MiSeq using 100 bp paired end reads. *E. coli* data was both mapped to the reference genome and *de novo* assembled. For the mapping analysis, libraries were mapped as single end reads to the reference *E. coli* K12 MG1655 genome using default Bowtie parameters. Contamination was analyzed, and clonal reads were removed using SAMtools' rmdup function. For the *de novo* assembly, paired end reads with a combined length less than 200 bp were first joined and treated as single end reads. All remaining paired end reads and newly generated single end reads were then quality trimmed. *De novo* assembly was performed using SPAdes¹¹ v. 2.4.0. Corrected reads were assembled with kmer values of 21, 33, and 55. The assembled scaffolds were mapped to the NCBI nt database with BLAST, and the organism distribution was visualized using MEGAN³⁵. Obvious contaminants (e.g., human) were removed from the assembly and the assembly was analyzed using QUAST³⁶. The remaining contigs were annotated using RAST³⁷ and KAAS³⁸.

### Example 1: MIDAS implements massively parallel polymerase cloning in microwells.

To implement "just-enough" amplification and thus limit the effects of the exponential amplification bias from MDA in a highly parallel manner, we designed and fabricated microwell arrays of a size comparable to standard microscope slides. The format of the microwell arrays, including well size, pattern, and spacing, was optimized to achieve efficient cell loading, optimal amplification yield, and convenient DNA extraction. Each slide contained 16 arrays each containing 156 microwells of 400µm in diameter, allowing for parallel amplification of 16 separate heterogeneous cell populations (**Fig. 1A**). All liquid handling procedures (cell seeding, lysis, DNA denaturation, neutralization and addition of amplification master mix) required only a single pipette pump per step per array, greatly reducing the labor required for hundreds of amplification reactions. The reagent cost is 1000-fold less than conventional methods, as each microwell is 12 nL in volume. In order to ensure that each reactor would contain only one single cell, we under-loaded the microwells at a density of roughly 1 cell per 10 wells, ensuring that no more than 0.5% of the wells would contain more than 1 cell. The remaining empty wells served as internal negative controls, allowing easy detection and elimination of contaminated samples. Proper microbial cell seeding in microwells was confirmed by scanning electron microscopy (**Fig**. **1B**).

After seeding of cell populations into each microwell array, we performed limited Multiple Displacement Amplification (MDA) on the seeded single cells at a reaction volume of ∼12nL in a temperature and humidity controlled chamber (**Fig. 1C**). We utilized SYBR Green I to visualize the amplicons growing in real-time using an epifluorescent microscope (**Fig. 5**). A random distribution of amplicons across the arrays was observed with approximately 10% of the wells containing amplicons, further confirming the parallel and localized amplification within individual microwells as well as the stochastic seeding of single cells²⁰. Exogenous contamination was easily detectible as a uniform increase of fluorescent signal across all microwells, allowing easy removal of contaminated samples. After amplification in the microwells, we employed a micromanipulation system to extract amplicons from individual wells for sequencing. (**Fig. 1C**). Fluorescent monitoring during this procedure ensured that only single wells were extracted for analysis (**Figs**. **6A**-**6B**). Using real-time MDA¹, we estimated that the extracted amplicon masses ranged from 500 picograms to 3 nanograms.

To construct Illumina sequencing libraries from the nanogram-scale DNA amplicons, we used a modified method based on the Nextera Tn5 transposase library construction kit. Previous studies have shown that Nextera transposase-based libraries can be prepared using as little as 10 picograms of genomic DNA²¹. However, the standard Nextera protocol was unable to generate high-complexity libraries from MDA amplicons, resulting in poor genomic coverage (data not shown). To address this issue, we used random hexamers and DNA Polymerase I to first convert the hyperbranched amplicons into unbranched double-stranded DNA molecules, which allowed effective library construction using the Nextera™ *in vitro* transposition method (**Fig. 1D**). We additionally used a small reaction volume to further increase the efficiency of the Nextera library construction²¹.

Thus, a sequencing library was constructed using products of substantially unbiased amplification in accordance with some examples herein.

### Example 2: MIDAS efficiently generates a near-complete genome assembly from single E. coli cells.

As a proof of concept, we utilized MIDAS on three single MG1655 *E. coli* cells and analyzed between approximately 2 - 8 million paired-end Illumina sequencing reads of 100 bp in length for each, which is equivalent to a genomic coverage of between 87x and 364x. We first mapped the reads to the reference *E. coli* genome and were able to recover between 94% and 99% of the genome at >1x coverage. We then performed *de novo* genome assembly using SPAdes²². We were able to assemble between 88% and 94% of the E. coli genome (**Fig. 2**), with an N50 contig size of 2,654 - 27,882 bp and a max contig length of 18,465 - 132,037 bp. More than 80% of the assembled bases were mapped to *E. coli,* with the remainder resulting from common MDA contaminants such as *Delftia* and *Acidovorax* **(****Fig. 7****, Table 1**). We annotated the genome using the RAST and KAAS annotation servers. Over 96% of *E. coli* genes were either partially or fully covered in the assembly. Major biosynthetic pathways, including glycolysis and the citric acid cycle, were also present. Furthermore, pathways for amino acid synthesis and tRNA development were covered. MIDAS was thus able to assemble an extremely large portion of the *E. coli* genome from a single cell with very minimal sequencing.

As a control, we also amplified and sequenced one *E. coli* cell using the conventional in-tube MDA method, and controlled the reaction time to limit the amplification yield to the nanogram level. A fraction of the control amplicon was further amplified in a second reaction to the microgram level. The two control amplicons were converted into sequencing libraries using the traditional shearing and ligation method. We found that limiting amplification yield resulted in a reduction of amplification bias even for in-tube amplification. However, MIDAS had a markedly reduced level of amplification bias when compared with either control reaction (**Figs. 3A-3D**). MIDAS was also able to recover a much larger fraction of the genome than the traditional MDA-based method. In fact, when compared with the most complete previously published single *E. coli* genome data set⁷, MIDAS was able to recover 50% more of the *E. coli* genome than the traditional MDA-based method with 3 to 13-fold less sequencing effort (∼90-400x vs. ∼1200x). This result demonstrates that MIDAS provides a much more efficient and cost-effective way to assemble whole bacterial genomes from single cells without culture.

### Example 3: MIDAS can identify small copy number variation in single human adult neurons.

Given the highly uniform genome coverage achieved by microwell based polymerase cloning, we next applied MIDAS to the characterization of copy number variation in single mammalian cells. The higher cognitive function of the human brain is supported by a complex network of neurons and glia. It has been long thought that all cells in a human brain share the same genome. Without being bound by any particular theory, recent evidence suggests that individual neurons could have non-identical genomes due to aneuploidy²³⁻²⁶, active retrotransposons^{27, 28} and other DNA content variation²⁹. However, the presence of somatic genetic variation in individual neurons has never been conclusively demonstrated at the single genome scale. To demonstrate the viability of MIDAS as a platform for investigating copy number variation in single primary human neurons, we prepared nuclei from one post-mortem brain sample from a healthy female donor and a second post-mortem brain sample from a female individual with Down Syndrome. We purified cortical neuronal nuclei by flow sorting based on neuron-specific NeuN antibody staining. Five sequencing libraries (two disease-free, three Down Syndrome) were generated from individual nuclei using MIDAS, and generated sequencing data was analyzed using an SNS method based on circular binary segmentation³⁰. We similarly observed a dramatic reduction of amplification bias in the MIDAS libraries when compared to the conventional in-tube MDA-based method (**Figs. 3C-D**).

We next sought to characterize the sensitivity of detecting single copy-number changes. While it was not possible, even with aggressive binning into large genomic regions, to distinguish true copy number differences from random amplification bias in the conventional single-cell MDA library, the uniform genome coverage in the MIDAS libraries allowed clear detection of Trisomy 21 in each of the Down Syndrome nuclei (**Fig. 4A**). Rigorous validation of single-cell sequencing methods has been extremely challenging, mainly due to the fact that any single cell analyzed might carry additional genomic differences from the bulk cell population. Hence, there is no reference genome that single cell data can be compared to. In order to determine the CNV detection limit of MIDAS, we computationally transplanted data from random 1 or 2 Mbps regions of either chromosome 21 (to simulate the gain of a single copy, the smallest possible copy number change) or chromosome 4 (as a negative control) from Down Syndrome nuclei into 100 other random genomic locations (**Table 2**). This computational approach, similar to a strategy previously used for assessing sequencing errors³¹, provided us a list of reference CNV events at various sizes for benchmarking without affecting the inherent technical noise in the data sets. We identified 68/100 (68%) of 1 Mb T21 insertions and 98/100 (98%) of 2 Mb T21 insertions, indicating that MIDAS is able to call copy number events at the megabase-scale with high sensitivity (**Fig**. **4B****, Table 2**). As expected, the insertion of diploid chromosome 4 regions did not generate any copy number calls. When the same simulation was performed with data from traditional in-tube MDA libraries, no T21 insertions were detected, indicating that at this level of sequencing depth, traditional MDA-based methods are unable to call small CNVs (**Figs. 8A-B**). We then performed CNV calling using the parameters calibrated by the T21 transplantation simulation. MIDAS additionally called 4-17 copy number events in each neuron (**Table 3**). Only 2/62 called CNV events were larger than 2 Mb, and 5/62 larger than 1 Mb. It remains unclear whether the remaining events represent true copy number changes or whether they are false positives due to the small size of most of the calls. However, five smaller CNV events were consistently called in two different nuclei from the healthy donor, and one additional CNV event on chromosome 10 was called in two nuclei from the Down Syndrome patient, suggesting that they are germ-line CNVs. Based on the T21 computational transplantation results, it appears that the five human neurons contain an average of 1 region each with 1 copy number gain at the megabase scale.

Thus, substantially unbiased amplification in accordance with some examples herein can sensitively detect changes in copy number of portions of a genome.

### Example 4: Identification of CNVs in MIDAS and MDA data

Mammalian single-cell libraries were sequenced in an Illumina Genome Analyzer IIx or Illumina HiSeq using 36 bp single end reads. The CNV algorithm previously published by Cold Spring Harbor Laboratories⁸ was used to call copy number variation on each single neuron, with modifications to successfully analyze non-cancer cells. Briefly, for each sample, reads were mapped to the genome using Bowtie. Clonal reads resulting from Polymerase Chain Reaction artifacts were removed using samtools, and the remaining unique reads were then assigned into 49,891 genomic bins that were previously determined such that each would contain a similar number of reads after mapping³⁰. Each bin's read count was then expressed as a value relative to the average number of reads per bin in the sample, and then normalized by GC content of each bin using a weighted sum of least squares algorithm (LOWESS). Circular binary segmentation was then used to divide each chromosome's bins into adjacent segments with similar means. Unlike the previously published algorithm, in which a histogram of bin counts was then plotted and the second peak chosen as representing a copy number of two, it was assumed, due to samples not being cancerous and thus being unlikely to contain significant amounts of aneuploidy, that the mean bin count in each sample would correspond to a copy number of two. Each segment's normalized bin count was thus multiplied by two and rounded to the nearest integer to call copy number. MIDAS data clearly showed a CNV call designating Trisomy 21 in all Down Syndrome single cells, while the traditional MDA-based method was not able to call Trisomy 21.

### Example 5: Identification of Artificial CNVs in MDA and MIDAS data

In order to test the ability of the CNV algorithm described above to call small CNVs, artificial CNVs were computationally constructed. Prior to circular binary segmentation, in each Down Syndrome sample, one hundred random genomic regions across chromosomes 1-22 were chosen, each consisting of either 17 or 34 bins of approximately 60 kb in size. Each region was replaced with an equivalently sized region from chromosome 21 or chromosome 4 (Supplementary Table 2). The above algorithm was then run on each "spiked-in" sample, and the number of new CNV calls in each sample that matched each spike-in was tallied. For the chromosome 21 spike-ins, MIDAS was able to accurately call 98% of spiked-in CNVs at the 2 Mb level and 68% of spiked-in CNVs at the 1 Mb level, while the traditional MDA-based method was not able to call any spiked-in CNVs. As expected, spike-ins of chromosome 4 did not result in any additional CNV calls.

Thus, small CNV's can be called in accordance with some examples herein.

**Table 1: Single E. coli assembly statistics**

| Total number of reads, number of contigs mapping to *E. coli,* N₅₀, maximum contig length, total base pairs assembled to *E. coli* K12 MG1655 genome, percent of *E. coli* K12 MG1655 covered in assembly, complete and partial genes covered, and percent of genome covered by mapped reads. Total number of reads refers to all sequencing reads, including non-mapping and clonal reads. | | | | | | | | |
|---|---|---|---|---|---|---|---|---|
| Cell No. | Total # of reads | # contigs greater than 500 bp | N₅₀ (bp) | Max contig (bp) | Total (bp) | % Genome Covered In Assembly | Complete/ Partial Genes Covered | % Genome Covered by Mapping |
| 1 | 2,019,892 | 1,172 | 6,416 | 32,552 | 4,283,777 | 92.33 | 3,308/ 775 | 97.85 |
| 2 | 3,884,950 | 2,102 | 2,654 | 18,465 | 4,065,096 | 87.62 | 2,313/ 1,683 | 93.81 |
| 3 | 8,482,573 | 765 | 27,882 | 132,037 | 4,368,254 | 94.15 | 3871 /185 | 98.71 |

**Table 2: Artificial CNV transplantation statistics.**

| Each genomic location used for calling of artificial CNVs is shown, along with whether or not MIDAS was able to call the artificial CNV. Only spike-ins of Trisomy Chromosome 21 from MIDAS samples generated CNV calls; spiking in either MIDAS Chromosome 4 or Trisomy Chromosome 21 from the traditional MDA-based method did not result in any artificial CNV calls. | | | | | | | |
|---|---|---|---|---|---|---|---|
| **2 Mb Spike-in Region** | **2Mb Spike-in Size** | **1 Mb Spike-in Region** | **1 Mb Spike-in Size** | **chr21 2 Mb Spike-in** | **chr21 1Mb Spike-in** | **2 Mb Spike -in Detec ted?** | **1 Mb Spik e-in Dete cted ?** |
| chr1:35,953,938-37,889,989 | 1,936,052 | chr1:35,953,938-36,992,975 | 1,039,038 | chr21:15,869,057-17,759,721 | chr21:15,869,057-16,841,316 | Yes | Yes |
| chr1:91,042,930-93,048,451 | 2,005,522 | chr1:91,042,930-92,070,940 | 1,028,011 | chr21:35,733,857-37,620,466 | chr21:35,733,857-36,687,022 | Yes | Yes |
| chr1:98,284,802-100,167,143 | 1,882,342 | chr1:98,284,802-99,236,989 | 952,188 | chr21:31,329,048-33,234,529 | chr21:31,329,048-32,284,116 | Yes | Yes |
| chr1:101,720,184-103,622,384 | 1,902,201 | chr1:101, 720,184-102,680,542 | 960,359 | chr21:15,549,571-17,439,036 | chr21:15,549,571-16,523,267 | Yes | Yes |
| chr1:158,948,121-160,904,574 | 1,956,454 | chr1:158, 948,121-159,956,586 | 1,008,466 | chr21:43,947,454-45,973,419 | chr21:43,947,454-45,032,873 | Yes | Yes |
| chr1:180,612,063-182,538,641 | 1,926,579 | chr1:180, 612,063-181,604,263 | 992,201 | chr21:18,144,565-20,109,045 | chr21:18,144,565-19,193,040 | Yes | No |
| chr1:219,167,316-221,099,50 4 | 1,932,189 | chr1:219, 167,316-220,161,764 | 994,449 | chr21:37,382,817-39,338,993 | chr21:37,382,817-38,415,585 | Yes | No |
| chr1:241,304,468-243,539,334 | 2,234,867 | chr1:241, 304,468-242,305,206 | 1,000,739 | chr21:45,256,736-47,160,835 | chr21:45,256,736-46,250,492 | Yes | Yes |
| chr2:47,279,743-49,257,602 | 1,977,860 | chr2:47,279,743-48,315,8 84 | 1,036,142 | chr21:43,895,354-45,919,088 | chr21:43,895,354-44,976,485 | Yes | Yes |
| chr2:51,016,978-52,900,279 | 1,883,302 | chr2:51,0 16,978-51,977,4 75 | 960,498 | chr21:28,485,490-30,475,416 | chr21:28,485,490-29,548,591 | Yes | Yes |
| chr2: 120,917,453-122,818,39 3 | 1,900,941 | chr2: 120,917,453-121,860, 157 | 942,705 | chr21:20,701,039-22,557,692 | chr21:20,701,039-21,663,245 | Yes | Yes |
| chr2: 139,284,812-141,151,575 | 1,866,764 | chr2:139,284,812-140,248,942 | 964,131 | chr21:21,715,029-23,559,945 | chr21:21,715,029-22,661,749 | No | No |
| chr2:151,537,791-153,484,681 | 1,946,891 | chr2:151,537,791-152,544,463 | 1,006,673 | chr21:19,016,794-20,913,230 | chr21:19,016,794-20,003,839 | Yes | No |
| chr2: 175,3 46,199-177,271,18 0 | 1,924,982 | chr2:175, 346,199-176,315, 579 | 969,381 | chr21:17,38 4,295-19,361,384 | chr21:17 ,38 4,295-18,362,470 | Yes | No |
| chr2:204,550,336-206,420,645 | 1,870,310 | chr2:204, 550,336-205,511, 965 | 961,630 | chr21:46,087,520-47,989,191 | chr21:46,087,520-47,051,166 | Yes | No |
| chr2:240,935,763-242,873,950 | 1,938,188 | chr2:240,935,763-241,911,231 | 975,469 | chr21:45,534,869-47,430,139 | chr21:45,534,869-46,517,024 | Yes | No |
| chr3:21,457,475-23,388,030 | 1,930,556 | chr3:21,457,475-22,429,886 | 972,412 | chr21:17,384,295-19,361,384 | chr21:17,384,295-18,362,470 | Yes | No |
| chr3:29,79 4,211-31,649,290 | 1,855,080 | chr3:29,7 94,211-30,766,2 80 | 972,070 | chr21:32,609,078-34,563,159 | chr21:32,609,078-33,620,107 | Yes | Yes |
| chr3:64,75 9,471-66,748,898 | 1,989,428 | chr3:64,7 59,471-65,729,4 10 | 969,940 | chr21:25,79 7,240-27,686,265 | chr21:25,79 7,240-26,755,429 | Yes | Yes |
| chr3:94,728,396-96,639,499 | 1,911,104 | chr3:94,728,396-95,718,3 72 | 989,977 | chr21:29,548,591-31,432,266 | chr21:29,548,591-30,529,736 | Yes | Yes |
| chr3: 131,350,124-133,321,647 | 1,971,524 | chr3:131,350,124-132,355,013 | 1,004,890 | chr21:20,380,269-22,240,402 | chr21:20,380,269-21,341,117 | Yes | Yes |
| chr3: 169,532,039-171,494,322 | 1,962,284 | chr3:169,532,039-170,559,996 | 1,027,958 | chr21:43,312,554-45,314,840 | chr21:43,312,554-44,279,997 | Yes | Yes |
| chr3:190,659,728-192,553,633 | 1,893,906 | chr3:190,659,728-191,648, 743 | 989,016 | chr21:45,699,913-47,592,766 | chr21:45,699,913-46,673,526 | Yes | No |
| chr4:26,71 7,043-28,591,712 | 1,874,670 | chr4:26,717,043-27,687,706 | 970,664 | chr21:34,563,159-36,473,184 | chr21:34,563,159-35,575,904 | Yes | No |
| chr4:41,807,132-43,732,453 | 1,925,322 | chr4:41,807,132-42,806,1 18 | 998,987 | chr21:46,19 5,641-48,129,895 | chr21:46,195,641-47,160,835 | Yes | No |
| chr4:47,152,041-52,814,683 | 5,662,643 | chr4:47,152,041-48,136,122 | 984,082 | chr21:41,811,953-43,737,990 | chr21:41,811,953-42,776,109 | Yes | Yes |
| chr4:55,03 6,501-56,991,161 | 1,954,661 | chr4:55,036,501-56,032,590 | 996,090 | chr21:38,137,201-40,021,631 | chr21:38,137,201-39,127,553 | Yes | Yes |
| chr4:59,922,675-61,851,268 | 1,928,594 | chr4:59,922,675-60,922,058 | 999,384 | chr21:41,100,954-43,045,476 | chr21:41,100,954-42,076,268 | Yes | Yes |
| chr4:62,174,303-64,098,264 | 1,923,962 | chr4:62,174,303-63,153,820 | 979,518 | chr21:39,759,243-41,648,348 | chr21:39,759,243-40,718,810 | Yes | Yes |
| chr4:68,752,406-70,866,031 | 2,113,626 | chr4:68,752,406-69,818,131 | 1,065,726 | chr21:20,965,576-22,821,144 | chr21:20,965,576-21,926,708 | Yes | Yes |
| chr4: 120,492,349-122,402,672 | 1,910,324 | chr4:120,492,349-121,477,609 | 985,261 | chr21:26,012,981-27,901,092 | chr21:26,012,981-26,970,320 | Yes | Yes |
| chr4:122,895,270-124,846,586 | 1,951,317 | chr4:122,895,270-123,899,166 | 1,003,897 | chr21:31,013,874-32,887,206 | chr21:31,013,874-31,960,666 | Yes | Yes |
| chr4:147,655,266-149,580,558 | 1,925,293 | chr4:147,655,266-148,648, 700 | 993,435 | chr21:33,344,236-35,307,614 | chr21:33,344,236-34,350,216 | Yes | Yes |
| chr5:42,837,955-44,872,058 | 2,034,104 | chr5:42,837,955-43,916,598 | 1,078,644 | chr21:25,960,034-27,848,628 | chr21 :25,960,034-26,918,132 | Yes | Yes |
| chr5:75,108,161-77,082,347 | 1,974,187 | chr5:75,108,161-76,129,009 | 1,020,849 | chr21:23,559,945-25,429,916 | chr21:23,559,945-24,529,659 | Yes | Yes |
| chr5:87,692,054-89,588,135 | 1,896,082 | chr5:87,692,054-88,655,990 | 963,937 | chr21:16,576,891-18,476,000 | chr21:16,576,891-17,549,158 | Yes | No |
| chr5:103,446,408-105,320,978 | 1,874,571 | chr5:103,446,408-104,415,213 | 968,806 | chr21:37,328,729-39,286,544 | chr21:37,328,729-38,356,392 | Yes | No |
| chr5:112,313,258-114,258,965 | 1,945,708 | chr5:112,313,258-113,297,312 | 984,055 | chr21:36,161,668-38,084,299 | chr21:36,161,668-37,109,297 | Yes | Yes |
| chr5:138,855,793-140,840,449 | 1,984,657 | chr5:138,855,793-139,867,090 | 1,011,298 | chr21:17,264,941-19,246,363 | chr21:17,264,941-18,248,286 | Yes | No |
| chr5:143,451,255-145,392,608 | 1,941,354 | chr5:143,451,255-144,448,400 | 997,146 | chr21:37,972,811-39,864,943 | chr21:37,972,811-38,971,124 | Yes | Yes |
| chr5:151,5 14,438-153,460,13 4 | 1,945,697 | chr5:151, 514,438-152,525, 509 | 1,011,072 | chr21:40,127,624-42,024,470 | chr21:40,127,624-41,100,954 | Yes | Yes |
| chr6:42,942,145-44,895,345 | 1,953,201 | chr6:42,942,145-43,948,4 91 | 1,006,347 | chr21:27,952,868-29,915,446 | chr21:27,952,868-28,908,233 | Yes | Yes |
| chr6:46,028,017-47,924,616 | 1,896,600 | chr6:46,0 28,017-46,996,5 71 | 968,555 | chr21:24,257,335-26,118,999 | chr21:24,257,335-25,216,223 | Yes | Yes |
| chr6:55,721,707 - 58,501,072 | 2,779,366 | chr6:55,721,707-56,691,195 | 969,489 | chr21:43,947,454-45,973,419 | chr21:43,947,454-45,032,873 | Yes | Yes |
| chr6:79,811,072-81,774,818 | 1,963,747 | chr6:79,811,072-80,802,629 | 991,558 | chr21:34,402,142-36,316,410 | chr21:34,402,142-35,416,887 | Yes | No |
| chr6:137,141,139-139,051,493 | 1,910,355 | chr6:137,141,139-138,109,997 | 968,859 | chr21:25,271,009-27,132,790 | chr21:25,271,009-26,223,918 | Yes | Yes |
| chr7:27,905,786-29,810,788 | 1,905,003 | chr7:27,905,786-28,850,6 65 | 944,880 | chr21:45,145,316-47,051,166 | chr21:45,145,316-46,142,620 | Yes | Yes |
| chr7:37,52 4,768-39,414,023 | 1,889,256 | chr7:37,524,768-38,513,924 | 989,157 | chr21:41,212,176-43,151,199 | chr21:41,212,176-42,183,940 | Yes | Yes |
| chr7:56,531,510-63,813,298 | 7,281,789 | chr7:56,5 31,510-62,248,786 | 5,717,277 | chr21:29,759,604-31,643,582 | chr21:29,759,604-30,743,560 | Yes | Yes |
| chr7: 131,3 71,835-133,239,40 3 | 1,867,569 | chr7:131, 371,835-132,316, 051 | 944,217 | chr21:42,23 6,377-44,163,506 | chr21:42,236,377-43,258,676 | Yes | Yes |
| chr7:142,172,461-144,654,928 | 2,482,468 | chr7:142,172,461-143,273,742 | 1,101,282 | chr21:14,820,139-17,160,267 | chr21:14,820,139-16,253,786 | Yes | Yes |
| chr8:31,002,063-32,894,131 | 1,892,069 | chr8:31,002,063-31,989,265 | 987,203 | chr21:39,864,943-41,758,932 | chr21:39,864,943-40,831,777 | No | No |
| chr8:58,142,435-60,031,197 | 1,888,763 | chr8:58,142,435-59,110,526 | 968,092 | chr21:42,076,268-43,998,204 | chr21:42,076,268-43,098,462 | Yes | Yes |
| chr8:78,929,030-80,820,600 | 1,891,571 | chr8:78,929,030-79,895,407 | 966,378 | chr21:18,248,286-20,218,830 | chr21:18,248,286-19,304,425 | Yes | No |
| chr9:1,055,886-2,968,704 | 1,912,819 | chr9:1,055,886-2,053,043 | 997,158 | chr21:45,145,316-47,051,166 | chr21:45,145,316-46,142,620 | Yes | Yes |
| chr9:26,653,725-28,577 ,848 | 1,924,124 | chr9:26,653,725-27,652,2 74 | 998,550 | chr21:45,200,702-47,107,423 | chr21:45,200,702-46,195,641 | Yes | Yes |
| chr9:78,438,145-80,383,381 | 1,945,237 | chr9:78,438,145-79,427,9 03 | 989,759 | chr21:29,602,048-31,485,060 | chr21:29,602,048-30,583,583 | Yes | Yes |
| chr9:85,352,360-87,347,753 | 1,995,394 | chr9:85,352,360-86,341,914 | 989,555 | chr21:36,473,184-38,415,585 | chr21:36,473,184-37,439,834 | Yes | Yes |
| chr9:108,943,484-110,868,83 1 | 1,925,348 | chr9:108,943,484-109,924, 645 | 981,162 | chr21:30,027,091-31,908,111 | chr21:30,027,091-31,013,874 | Yes | No |
| chr9:136,054,731-138,033,870 | 1,979,140 | chr9:136,054,731-137,134, 437 | 1,079,707 | chr21:27,357,146-29,249,735 | chr21:27,357,146-28,326,314 | Yes | No |
| chr9:138,851,087-140,884,555 | 2,033,469 | chr9:138,851,087-139,905,741 | 1,054,655 | chr21:41,432,175-43,366,620 | chr21:41,432,175-42,401,776 | Yes | Yes |
| chr10:37,235,643-43,386,249 | 6,150,607 | chr10:37,235,643-38,367,6 12 | 1,131,970 | chr21:29,438,499-31,329,048 | chr21:29,438,499-30,420,106 | Yes | Yes |
| chr10:50,517,442-53,244,584 | 2,727,143 | chr10:50, 517,442-52,259,071 | 1,741,630 | chr21:41,265,140-43,202,177 | chr21:41,265,140-42,236,377 | Yes | Yes |
| chr10:84,733,418-86,669,139 | 1,935,722 | chr10:84,733,418-85,709,427 | 976,010 | chr21:30,027,091-31,908,111 | chr21:30,027,091-31,013,874 | Yes | Yes |
| chr11:1,155,181-3,103,945 | 1,948,765 | chr11:1,155,181-2,197,444 | 1,042,264 | chr21:32,662,136-34,618,144 | chr21:32,662,136-33,675,918 | Yes | Yes |
| cbr11:60,102,184-62,149,657 | 2,047,474 | chr11:60,102,184-61,169,073 | 1,066,890 | chr21:33,454,741-35,416,887 | chr21:33,454,741-34,454,555 | Yes | No |
| chr11:87,270,689-89,838,290 | 2,567,602 | chr11:87,270,689-88,321,4 83 | 1,050,795 | chr21:27,572,323-29,548,591 | chr21:27,572,323-28,537,710 | Yes | No |
| chr11:90,9 97,546-92,889,388 | 1,891,843 | chr11:90, 997,546-91,975,9 42 | 978,397 | chr21:38,031,056-39,916,751 | chr21:38,031,056-39,023,029 | Yes | No |
| chr11:109,670,892-111,573,136 | 1,902,245 | chr11:10 9,670,89 2-110,675, 346 | 1,004,455 | chr21:32,174,455-34,132,773 | chr21:32,174,455-33,174,429 | Yes | No |
| chr11:116,094,960-118,005,159 | 1,910,200 | chr11:116,094,960-117,087, 728 | 992,769 | chr21:21,017,554-22,872,356 | chr21:21,017,554-21,979,621 | Yes | No |
| chr11:125,026,409-126,921,222 | 1,894,814 | chr11:125,026,409-126,022, 156 | 995,748 | chr21:28,695,036-30,689,040 | chr21:28,695,036-29,759,604 | Yes | No |
| chr12:49,227,100-51,283,876 | 2,056,777 | chr12:49,227,100-50,281,689 | 1,054,590 | chr21:45,145,316-47,051,166 | chr21:45,145,316-46,142,620 | Yes | Yes |
| chr12:52,0 63,249-54,036,936 | 1,973,688 | chr12:52, 063,249-53,042,314 | 979,066 | chr21:44,467,407-46,465,237 | chr21:44,467,407-45,534,869 | Yes | Yes |
| chr12:93,762,836-95,714,509 | 1,951,674 | chr12:93,762,836-94,755,100 | 992,265 | chr21:22,083,898-23,935,965 | chr21:22,083,898-23,028,045 | Yes | No |
| chr12:105,555,128-107,478,702 | 1,923,575 | chr12:105,555,128-106,536,849 | 981,722 | chr21:34,509,021-36,421,675 | chr21:34,509,021-35,521,899 | Yes | Yes |
| chr12: 125,199,005-127,114,720 | 1,915,716 | chr12:125,199,005-126,193,374 | 994,370 | chr21:41,26 5,140-43,202,177 | chr21:41,265,140-42,236,377 | Yes | Yes |
| chr13:20,265,706-22,292,811 | 2,027,106 | chr13:20,265,706-21,295,812 | 1,030,107 | chr21:21,286,351-23,135,668 | chr21:21,286,351-22,240,402 | Yes | Yes |
| chr13:72,352,465-74,251,208 | 1,898,744 | chr13:72,352,465-73,311,492 | 959,028 | chr21:32,067,733-34,023,166 | chr21:32,067,733-33,061,883 | Yes | No |
| chr13:111,996,474-114,071,865 | 2,075,392 | chr13:111,996,474-113,130,054 | 1,133,581 | chr21:30,475,416-32,337,385 | chr21:30,475,416-31,432,266 | Yes | Yes |
| chr14:40,383,400-42,297,026 | 1,913,627 | chr14:40, 383,400-41,368,4 53 | 985,054 | chr21:16,421,666-18,309,003 | chr21:16,421,666-17,384,295 | Yes | No |
| chr14:47,205,765-49,129,974 | 1,924,210 | chr14:47,205,765-48,164,1 35 | 958,371 | chr21:36,842,693-38,809,659 | chr21:36,842,693-37,864,928 | Yes | No |
| chr14:49,785,308-51,764,266 | 1,978,959 | chr14:49,785,308-50,827,716 | 1,042,409 | chr21:34,618,144-36,526,570 | chr21:34,618,144-35,629,603 | Yes | No |
| chr14:54,010,611-55,940,946 | 1,930,336 | chr14:54,010,611-54,992,372 | 981,762 | chr21:16,31,320-18,197,321 | chr21: 16,317,320-17,264,941 | Yes | Yes |
| chr14:58,280,794-60,215,080 | 1,934,287 | chr14:58,280,794-59,282,961 | 1,002,168 | chr21:20,808,528-22,661,749 | chr21:20,808,528-21,768,367 | Yes | Yes |
| chr14:65,798,341-67,757,790 | 1,959,450 | chr14:65,798,341-66,798,253 | 999,913 | chr21:45,087,470-46,993,898 | chr21:45,08 7,470-46,087,520 | Yes | Yes |
| chr14:69,993,123-71,950,871 | 1,957,749 | chr14:69,993,123-70,999,0 14 | 1,005,892 | chr21:40,718,810-42,616,819 | chr21:40,718,810-41,704,149 | Yes | Yes |
| chr14:79,855,300-81,757,060 | 1,901,761 | chr14:79,855,300-80,828,708 | 973,409 | chr21:26,970,320-28,855,428 | chr21:26,970,320-27,952,868 | Yes | Yes |
| chr15:34,986,155-36,878,959 | 1,892,805 | chr15:34,986,155-35,976,285 | 990,131 | chr21:31,643,582-33,561,518 | chr21:31,643,582-32,609,078 | Yes | Yes |
| chr15:79,683,541-81,583,565 | 1,900,025 | chr15:79,683,541-80,679,599 | 996,059 | chr21:40,551,918-42,454,621 | chr21:40,551,918-41,542,378 | Yes | Yes |
| chr16:47,776,123-49,696,183 | 1,920,061 | chr16:47,776,123-48,790,979 | 1,014,857 | chr21:40,127,624-42,024,470 | chr21:40,127,624-41,100,954 | Yes | No |
| chr16:73,420,082-75,485,559 | 2,065,478 | chr16:73,420,082-74,472,527 | 1,052,446 | chr21:23,935,965-25,797,240 | chr21 :23,935,965-24,896,759 | Yes | Yes |
| chr17:4,089,647-6,142,294 | 2,052,648 | chr17:4,089,647-5,192,301 | 1,102,655 | chr21:36,421,675-38,356,392 | chr21:36,421,675-37,382,817 | Yes | Yes |
| chr17:13,450,820-15,379,070 | 1,928,251 | chr17:13,450,820-14,460,546 | 1,009,727 | chr21:46,195,641-48,129,895 | chr21:46,195,641-47,160,835 | Yes | Yes |
| chr17:37,373,102-39,387,990 | 2,014,889 | chr17:37,373,102-38,433,119 | 1,060,018 | chr21:35,307,614-37,163,621 | chr21:35,307,614-36,264,921 | Yes | Yes |
| chr17:42,946,627 - 45,535,886 | 2,589,260 | chr17:42,946,627-44,092,717 | 1,146,091 | chr21:39,023,029-40,887,012 | chr21:39,023,029-39,970,006 | Yes | Yes |
| chr17:62,034,684-64,166,903 | 2,132,220 | chr17:62,034,684-63,235,801 | 1,201,118 | chr21:23,770,888-25,639,505 | chr21:23,770,888-24,739,139 | Yes | Yes |
| chr18:42,248,309-44,139,666 | 1,891,358 | chr18:42,248,309-43,192,657 | 944,349 | chr21:36,900,623-38,861,882 | chr21:36,900,623-37,919,721 | Yes | No |
| chr19:24,366,238-29,680,778 | 5,314,541 | chr19:24,366,238-28,755,127 | 4,388,890 | chr21:20,965,576-22,821,144 | chr21:20,965,576-21,926,708 | Yes | Yes |
| chr20:47,231,329-49,203,437 | 1,972,109 | chr20:47,231,329-48,237,944 | 1,006,616 | chr21:16,630,136-18,541,644 | chr21:16,630,136-17,604,225 | Yes | Yes |
| chr22:49,539,479-51,909,988 | 2,370,510 | chr22:49,539,479-50,562,673 | 1,023,195 | chr21:30,361,934-32,231,305 | chr21:30,361,934-31,329,048 | Yes | Yes |

**Table 3: Copy number events called in each neuron.**

| All identified copy number events in each single cell are listed, along with the size of the CNV in actual base pairs and number of base pairs in the CNV that were non-repetitive according to a previously published algorithm ⁸. Unique CNVs are presented in plain text, while CNVs shared between one or more samples are presented in *italics* (if a CNV call was partially identified in another sample) or **bold** (if a CNV call was fully identified in another sample). Aside from Trisomy 21 (identified in all three Down Syndrome cells), most CNV calls are fairly small in both size and non-repetitive size. | | | | | | | |
|---|---|---|---|---|---|---|---|
| **Cell #** | **Type** | **Chromosome** | **Start Position** | **End Position** | **Copy No.** | **Size** | **Size (Valid Genomic Regions⁸)** |
| **1** | **Healthy** | **1** | **16,949,551** | **17,257,431** | **5** | **307,881** | **120,000** |
| **1** | **Healthy** | **1** | **147,802,093** | **149,049,044** | **3** | **1,246,952** | **120,000** |
| *1* | *Healthy* | *2* | *133,000,723* | *133,135,043* | *4* | *134,321* | *120,000* |
| *1* | Healthy | 3 | 75,275,861 | 76,035,772 | 3 | 759,912 | 420,000 |
| *1* | *Healthy* | *4* | *190,664,845* | *191,154,276* | *4* | *489,432* | *240,000* |
| *1* | *Healthy* | *6* | *32,526,395* | *32,645,736* | *1* | *119,342* | *120,000* |
| *1* | *Healthy* | *8* | *39,308,029* | *39,363,306* | *1* | *55,278* | *60,000* |
| **1** | **Healthy** | **10** | **47,008,316** | **47,538,599** | **4** | **530,284** | **180,000** |
| 1 | Healthy | 11 | 48,858,583 | 48,959,202 | 4 | 100,620 | 60,000 |
| 1 | Healthy | 11 | 122,887,817 | 123,010,937 | 1 | 123,121 | 120,000 |
| **1** | **Healthy** | **15** | **34,761,777** | **34,873,738** | **1** | **111,962** | **60,000** |
| 1 | Healthy | 16 | 3,762,009 | 3,818,563 | 1 | 56,555 | 60,000 |
| 1 | Healthy | 16 | 32,340,630 | 34,746,226 | 3 | 2,405,597 | 1,140,000 |
| *1* | *Healthy* | *16* | *71,141,287* | *71,246,392* | *7* | *105,106* | *60,000* |
| **1** | **Healthy** | **17** | **21,257,685** | **21,374,155** | **3** | **116,471** | **120,000** |
| 1 | Healthy | 17 | 77,452,319 | 77,652,085 | 4 | 199,767 | 60,000 |
| *1* | *Healthy* | *20* | *29,449,066* | *29,811,435* | *4* | *362,370* | *120,000* |
| **2** | **Healthy** | **1** | **16,949,551** | **17,257,431** | **4** | **307,881** | **120,000** |
| 2 | Healthy | 1 | 34,347,191 | 34,666,699 | 3 | 319,509 | 360,000 |
| **2** | **Healthy** | **1** | **147,802,093** | **149,049,044** | **4** | **1,246,952** | **120,000** |
| 2 | Healthy | 2 | 132,846,449 | 133,135,043 | 3 | 288,595 | 180,000 |
| 2 | Healthy | 3 | 75,803,231 | 75,901,346 | 4 | 98,116 | 60,000 |
| 2 | Healthy | 3 | 195,457,070 | 195,525,025 | 3 | 67,956 | 60,000 |
| 2 | Healthy | 6 | 0 | 358,119 | 3 | 358,120 | 180,000 |
| *2* | *Healthy* | *6* | *32,526,395* | *32,699,933* | *1* | *173,539* | *180,000* |
| *2* | *Healthy* | *8* | *39,308,029* | *39,363,306* | *1* | *55,278* | *60,000* |
| **2** | **Healthy** | **10** | **47,008,316** | **47,538,599** | **4** | **530,284** | **180,000** |
| **2** | **Healthy** | **15** | **34,761,777** | **34,873,738** | **1** | **111,962** | **60,000** |
| 2 | Healthy | 16 | 32,499,141 | 34,280,003 | 3 | 1,780,863 | 600,000 |
| 2 | Healthy | 16 | 34,410,499 | 34,746,226 | 3 | 335,728 | 360,000 |
| *2* | *Healthy* | *16* | *71,141,287* | *71,246,392* | *9* | *105,106* | *60,000* |
| **2** | **Healthy** | **17** | **21,257,685** | **21,374,155** | **3** | **116,471** | **120,000** |
| 2 | Healthy | 18 | 59,103,041 | 59,431,597 | 3 | 328,557 | 360,000 |
| 2 | Healthy | 20 | 25,753,877 | 29,868,184 | 3 | 4,114,308 | 420,000 |
| 2 | Healthy | 20 | 35,971,800 | 36,129,265 | 3 | 157,466 | 180,000 |
| *3* | *Down Syndrome* | *4* | *190,664,845* | *191,154,276* | *4* | *489,432* | *240,000* |
| *3* | *Down Syndrome* | *8* | *39,308,029* | *39*,*363*,*306* | *0* | *55,278* | *60,000* |
| **3** | **Down Syndrome** | **10** | **38,869,769** | **42,858,972** | **6** | **3,989,204** | **240,000** |
| 3 | Down Syndrome | 10 | 47,008,316 | 50,466,755 | 3 | 3,458,440 | 2,040,000 |
| 3 | Down Syndrome | 10 | 69,854,431 | 70,514,102 | 1 | 659,672 | 660,000 |
| *3* | *Down Syndrome* | *16* | *71,141,287* | *71,246,392* | *11* | *105,106* | *60,000* |
| 3 | Down Syndrome | 19 | 31,729,973 | 32,514,241 | 1 | 784,269 | 900,000 |
| *3* | *Down Syndrome* | *20* | *29,449,066* | *29,868,184* | *6* | *419,119* | *180,000* |
| 3 | Down Syndrome | 20 | 42,392,899 | 43,933,855 | 3 | 1,540,957 | 1,680,000 |
| **3** | **Down Syndrome** | **21** | **14,432,540** | **48,129,895** | **3** | **33,697,356** | **36,180,000** |
| 3 | Down Syndrome | 22 | 50,785,685 | 51,304,566 | 1 | 518,882 | 420,000 |
| 4 | Down Syndrome | 1 | 114,955,315 | 115,008,910 | 1 | 53,596 | 60,000 |
| *4* | *Down Syndrome* | *2* | *133,000,723* | *133,065,776* | *39* | *65*,*054* | *60,000* |
| 4 | Down Syndrome | 3 | 180,472,228 | 180,582,904 | 0 | 110,677 | 120,000 |
| 4 | Down Syndrome | 4 | 68,807,337 | 68,866,453 | 0 | 59,117 | 60,000 |
| 4 | Down Syndrome | 4 | 107,214,750 | 107,376,615 | 0 | 161,866 | 180,000 |
| *4* | *Down Syndrome* | *8* | *39,308,029* | *39,415,337* | *0* | *107,309* | *120,000* |
| **4** | **Down Syndrome** | **10** | **38,869,769** | **42,858,972** | **8** | **3,989,204** | **240,000** |
| 4 | Down Syndrome | 10 | 61,755,833 | 61,810,282 | 1 | 54,450 | 60,000 |
| 4 | Down Syndrome | 10 | 65,820,124 | 67,011,981 | 3 | 1,191,858 | 1,320,000 |
| 4 | Down Syndrome | 16 | 34,002,234 | 34,220,262 | 7 | 218,029 | 60,000 |
| 4 | Down Syndrome | 19 | 29,082,056 | 29,963,452 | 3 | 881,397 | 960,000 |
| 4 | Down Syndrome | 19 | 53,713,097 | 54,229,102 | 1 | 516,006 | 480,000 |
| *4* | *Down Syndrome* | *20* | *29,449,066* | *29,927,709* | *6* | *478,644* | *240,000* |
| **4** | **Down Syndrome** | **21** | **14,432,540** | **48,129,895** | **3** | **33,697,356** | **36,180,000** |
| 5 | Down Syndrome | 10 | 12,083,581 | 12,397,568 | 1 | 313,988 | 300,000 |
| **5** | **Down Syndrome** | **10** | **38,869,769** | **42,858,972** | **9** | **3,989,204** | **240,000** |
| 5 | Down Syndrome | 20 | 21,609,652 | 23,522,517 | 3 | 1,912,866 | 2,100,000 |
| *5* | *Down Syndrome* | *20* | *29,449,066* | *29,868,184* | *9* | *419,119* | *180,000* |
| **5** | **Down Syndrome** | **21** | **14,432,540** | **48,129,895** | **3** | **33,697,356** | **36,180,000** |

### References

1. Zhang, K. et al. Sequencing genomes from single cells by polymerase cloning. Nat Biotechnol 24, 680-686 (2006).
2. Rodrigue, S. et al. Whole genome amplification and de novo assembly of single bacterial cells. PLoS One 4, e6864 (2009).
3. Fan, H.C., Wang, J., Potanina, A. & Quake, S.R. Whole-genome molecular haplotyping of single cells. Nat Biotechnol 29, 51-57 (2011).
4. Hou, Y. et al. Single-cell exome sequencing and monoclonal evolution of a JAK2-negative myeloproliferative neoplasm. Cell 148, 873-885 (2012).
5. Pan, X. et al. A procedure for highly specific, sensitive, and unbiased whole-genome amplification. Proc Natl Acad Sci USA 105, 15499-15504 (2008).
6. Marcy, Y. et al. Dissecting biological "dark matter" with single-cell genetic analysis of rare and uncultivated TM7 microbes from the human mouth. Proc Natl Acad Sci U S A 104, 11889-11894 (2007).
7. Yoon, H.S. et al. Single-cell genomics reveals organismal interactions in uncultivated marine protists. Science 332, 714-717 (2011).
8. Navin, N. et al. Tumour evolution inferred by single-cell sequencing. Nature 472, 90-94 (2011).
9. Xu, X. et al. Single-cell exome sequencing reveals single-nucleotide mutation characteristics of a kidney tumor. Cell 148, 886-895 (2012).
10. Wang, J., Fan, H.C., Behr, B. & Quake, S.R. Genome-wide single-cell analysis of recombination activity and de novo mutation rates in human sperm. Cell 150, 402-412 (2012).
11. Bankevich, A. et al. SPAdes: a new genome assembly algorithm and its applications to single-cell sequencing. J Comput Biol 19, 455-477 (2012).
12. Chitsaz, H. et al. Efficient de novo assembly of single-cell bacterial genomes from short-read data sets. Nat Biotechnol 29, 915-921 (2011).
13. Hutchison, C.A., 3rd, Smith, H.O., Pfannkoch, C. & Venter, J.C. Cell-free cloning using phi29 DNA polymerase. Proc Natl Acad Sci USA 102, 17332-17336 (2005).
14. Marcy, Y. et al. Nanolitre reactors improve multiple displacement amplification of genomes from single cells. PLoS Genet 3, 1702-1708 (2007).
15. Inoue, J., Shigemori, Y. & Mikawa, T. Improvements of rolling circle amplification (RCA) efficiency and accuracy using Thermus thermophilus SSB mutant protein. Nucleic Acids Res 34, e69 (2006).
16. Pan, X. et al. A procedure for highly specific, sensitive, and unbiased whole-genome amplification. Proc Natl Acad Sci USA 105, 15499-15504 (2008).
17. Rodrigue, S. et al. Whole genome amplification and de novo assembly of single bacterial cells. PLoS One 4, e6864 (2009).
18. Woyke, T. et al. One bacterial cell, one complete genome. PLoS One 5, e10314 (2010).
19. Fitzsimons, M.S. et al. Nearly finished genomes produced using gel microdroplet culturing reveal substantial intraspecies genomic diversity within the human microbiome. Genome Res (2013).
20. Blainey, P.C. & Quake, S.R. Digital MDA for enumeration of total nucleic acid contamination. Nucleic acids research 39, e19 (2011).
21. Adey, A. & Shendure, J. Ultra-low-input, tagmentation-based whole-genome bisulfite sequencing. Genome Res 22, 1139-1143 (2012).
22. Bankevich, A. et al. SPAdes: a new genome assembly algorithm and its applications to single-cell sequencing. J Comput Biol 19, 455-477 (2012).
23. Rehen, S.K. et al. Constitutional aneuploidy in the normal human brain. J Neurosci 25, 2176-2180 (2005).
24. Rehen, S.K. et al. Chromosomal variation in neurons of the developing and adult mammalian nervous system. Proc Natl Acad Sci U S A 98, 13361-13366 (2001).
25. Yang, A.H. et al. Chromosome segregation defects contribute to aneuploidy in normal neural progenitor cells. J Neurosci 23, 10454-10462 (2003).
26. Yurov, Y.B. et al. Aneuploidy and confined chromosomal mosaicism in the developing human brain. PLoS One 2, e558 (2007).
27. Muotri, A.R. & Gage, F.H. Generation of neuronal variability and complexity. Nature 441, 1087-1093 (2006).
28. Singer, T., McConnell, M.J., Marchetto, M.C., Coufal, N.G. & Gage, F.H. LINE-1 retrotransposons: mediators of somatic variation in neuronal genomes? Trends Neurosci 33, 345-354 (2010).
29. Westra, J.W. et al. Neuronal DNA content variation (DCV) with regional and individual differences in the human brain. J Comp Neurol 518, 3981-4000 (2010).
30. Baslan, T. et al. Genome-wide copy number analysis of single cells. Nat Protoc 7, 1024-1041 (2012).
31. Shendure, J. et al. Accurate multiplex polony sequencing of an evolved bacterial genome. Science 309, 1728-1732 (2005).
32. Zong, C., Lu, S., Chapman, A.R. & Xie, X.S. Genome-wide detection of single-nucleotide and copy-number variations of a single human cell. Science 338, 1622-1626 (2012).
33. Hussein, S.M. et al. Copy number variation and selection during reprogramming to pluripotency. Nature 471, 58-62 (2011).
34. Westra, J.W. et al. Aneuploid mosaicism in the developing and adult cerebellar cortex. J Comp Neurol 507, 1944-1951 (2008).
35. Huson, D.H., Auch, A.F., Qi, J. & Schuster, S.C. MEGAN analysis of metagenomic data. Genome Res 17, 377-386 (2007).
36. Gurevich, A., Saveliev, V., Vyahhi, N. & Tesler, G. QUAST: quality assessment tool for genome assemblies. Bioinformatics 29, 1072-1075 (2013).
37. Aziz, R.K. et al. The RAST Server: rapid annotations using subsystems technology. BMC Genomics 9, 75 (2008).
38. Moriya, Y., Itoh, M., Okuda, S., Yoshizawa, A.C. & Kanehisa, M. KAAS: an automatic genome annotation and pathway reconstruction server. Nucleic acids research 35, W182-185 (2007).
39. Fan, Christina et al. Whole genome molecular haplotyping of single cells Nature Biotech
40. Zhong, Chenghang et al. Genome-Wide Detection of Single-Nucleotide and Copy-Number Variations of a Single Human Cell Science 338 1622 (2012)
41. Zhang, Kun et al. Sequencing Genomes from Single Cells by Polymerase Cloning Nature Biotech
42. Evrony, Gilrad et al. Single Neuron Sequencing Analysis of L1 Retrotransposition and Somatic Mutation in the Human Brain Cell 151 483 (2012)
43. Kirkness, E.F. et al. Sequencing of isolated sperm cells for direct haplotyping of a human genome. Genome Res. 23, 826-832 (2013).
44. Lu, S. et al. Probing meiotic recombination and aneuploidy of single sperm cells by whole-genome sequencing. Science 338, 1627-1630 (2012).

In this application, the use of the singular can include the plural unless specifically stated otherwise or unless, as will be understood by one of skill in the art in light of the present disclosure, the singular is the only functional embodiment. Thus, for example, "a" can mean more than one, and "one embodiment" can mean that the description applies to multiple embodiments.

## Claims

1. A method of producing a substantially unbiased amplification library of a genome of a single cell, the method comprising:
- applying a liquid comprising diluted whole cells or fractions thereof and the single cell to a substrate comprising a plurality of nanolitre-scale reaction environments each having a volume of 1 nanolitre to 50 nanolitres, wherein the single cell is in a nanolitre-scale reaction environment of the plurality of reaction environments, and wherein at least 95% of the nanolitre-scale reaction environments do not comprise any genomes other than the genome of the single cell;
- amplifying, by multiple strand displacement amplification, the genome of the single cell in the nanolitre-scale reaction environment, wherein a ratio of amount of nucleic acid of the genome to volume of the nanolitre-scale reaction environment is at least 0.3 Mega-basepairs per nanolitre, and wherein the nanolitre-scale reaction environment is configured for substantially unbiased amplification of the genome, said amplifying comprising contacting the reaction environment with (a) strand-displacement polymerase, and (b) a plurality of random multimers of DNA, wherein said amplifying is performed until additional cycles of amplification are no longer in a logarithmic phase, thereby producing a plurality of amplicons of a substantially unbiased amplification of the genome; and
- constructing a library comprising a plurality of amplicons of the substantially unbiased amplification of the genome.

2. The method of claim 1, wherein the nanolitre-scale reaction environment is configured for amplification of at least about 90% of the genome at greater than 1x coverage.

3. The method of any one of claims 1-2, wherein the nanolitre-scale reaction environment comprises a volume of no more than about 20nL.

4. The method of any one of claims 1-3, further comprising amplifying a plurality of genomes of single cells in the plurality of nanolitre-scale reaction environments on a single substrate, wherein at least 95% of the reaction environments do not comprise any genomes other than a genome of a single cell.

5. The method of claim 4, further comprising identifying a reaction environment in which a desired level of amplification has been achieved, wherein the library is constructed from the reaction environment in which a desired level of amplification has been achieved.

6. The method of any one of claims 4-5, further comprising constructing a plurality of libraries from the plurality of reaction environments, wherein the number of the plurality of libraries is the same or different as the number of the plurality of reaction environments.

7. The method of any one of claims 1-6, wherein amplifying the genome of the single cell in the nanolitre-scale reaction environment comprises amplification in the presence of an amplification-detection moiety, and wherein signal from the amplification-detection moiety identifies a reaction environment in which a desired level of amplification has been achieved.

8. The method of any one of claims 1-7, wherein the reaction environment does not comprise any genomes other than the genome of the single cell.

9. The method of any one of claims 1-8, wherein substantially all of the plurality of amplicons are unbranched.

10. The method of any one of claims 1-9, wherein the plurality of amplicons comprises no more than about 100 picograms to about 10 nanograms of DNA.

11. The method of any one of the above claims, wherein the method is performed in parallel on two or more genomes of two or more single cells, thereby producing two or more unbiased amplification libraries in parallel.

12. A method of producing a substantially unbiased amplification of a genome by multiple strand displacement amplification (MDA), the method comprising:
- applying a liquid comprising diluted whole cells or fractions thereof to a substrate comprising a nanolitre-scale reaction environment having a volume of 1 nanolitre to 50 nanolitres, wherein there is at least a 99% probability that the nanolitre-scale reaction environment comprises no more than one genome, wherein a ratio of amount of nucleic acid of the genome to volume of the nanolitre-scale reaction environment is at least 0.3 Mega-basepairs per nanolitre; and
- contacting the nanolitre-scale reaction environment with (a) strand-displacement polymerase, and (b) a plurality of random multimers of DNA, and amplifying the genome by MDA until additional cycles of amplification are no longer in a logarithmic phase, thereby producing a substantially unbiased amplification of the genome.

13. The method of claim 12, further comprising constructing a library comprising a plurality of amplicons of the substantially unbiased amplification of the genome.

14. The method of any one of claims 1-11, said applying comprising spreading the plurality of cells evenly across a loading areas of the substrate, whereby at least 99% of the plurality of nanolitre-scale reaction environments contain no more than 1 cell per well.

## Patentansprüche

1. Verfahren zur Erzeugung einer im Wesentlichen verzerrungsfreien Amplifikationsbibliothek eines Genoms einer einzelnen Zelle, wobei das Verfahren folgendes umfasst:
- Applizieren einer Flüssigkeit, die verdünnte Ganzzellen oder Teile dieser und die einzelne Zelle umfasst, auf ein Substrat, das mehrere Reaktionsumgebungen im Nanolitermaßstab umfasst, die jeweils ein Volumen von 1 Nanoliter bis 50 Nanolitern aufweisen, wobei sich die einzelne Zelle in einer Reaktionsumgebung im Nanolitermaßstab der mehreren Reaktionsumgebungen befindet, und wobei wenigstens 95% der Reaktionsumgebungen im Nanolitermaßstab keine anderen Genome als das Genom der einzelnen Zelle umfassen;
- Amplifizieren des Genoms der einzelnen Zelle in der Reaktionsumgebung im Nanolitermaßstab durch Multiple-Strand-Displacement-Amplifikation, wobei ein Verhältnis der Nukleinsäuremenge des Genoms zu dem Volumen der Reaktionsumgebung im Nanolitermaßstab wenigstens 0,3 Megabasenpaare pro Nanoliter beträgt, und wobei die Reaktionsumgebung im Nanolitermaßstab für eine im Wesentlichen verzerrungsfreie Amplifikation des Genoms gestaltet ist, wobei das Amplifizieren das Inkontaktbringen der Reaktionsumgebung mit (a) Strand-Displacement-Polymerase und (b) einer Mehrzahl wahlfreier Multimere von DNA umfasst, wobei das Amplifizieren durchgeführt wird, bis zusätzliche Amplifikationszyklen nicht mehr in einer logarithmischen Phase sind, wodurch eine Mehrzahl von Amplicons einer im Wesentlichen verzerrungsfreien Amplifikation des Genoms erzeugt wird; und
- Aufbauen einer Bibliothek, die eine Mehrzahl von Amplicons der im Wesentlichen verzerrungsfreien Amplifikation des Genoms umfasst.

2. Verfahren nach Anspruch 1, wobei die Reaktionsumgebung im Nanolitermaßstab für eine Amplifikation von mindestens etwa 90% des Genoms bei einer Abdeckung von mehr als 1x gestaltet ist.

3. Verfahren nach Anspruch 1 oder 2, wobei die Reaktionsumgebung im Nanolitermaßstab ein Volumen von nicht mehr als etwa 20 nL umfasst.

4. Verfahren nach einem der Ansprüche 1 bis 3, ferner umfassend das Amplifizieren einer Mehrzahl von Genomen einzelner Zellen in der Mehrzahl von Reaktionsumgebungen im Nanolitermaßstab auf einem einzelnen Substrat, wobei wenigstens 95% der Reaktionsumgebungen keine anderen Genome als ein Genom einer einzelnen Zelle umfassen.

5. Verfahren nach Anspruch 4, ferner umfassend das Identifizieren einer Reaktionsumgebung, in der ein gewünschtes Maß der Amplifikation erreicht worden ist, wobei die Bibliothek aus der Reaktionsumgebung aufgebaut wird, in der ein gewünschtes Maß der Amplifikation erreicht worden ist.

6. Verfahren nach Anspruch 4 oder 5, ferner umfassend das Aufbauen mehrerer Bibliotheken aus der Mehrzahl von Reaktionsumgebungen, wobei die Anzahl der Mehrzahl von Bibliotheken gleich oder unterschiedlich der Anzahl der Mehrzahl von Reaktionsumgebungen ist.

7. Verfahren nach einem der Ansprüche 1 bis 6, wobei das Amplifizieren des Genoms der einzelnen Zelle in der Reaktionsumgebung im Nanolitermaßstab die Amplifikation in Gegenwart eines Amplifikationsnachweisteils umfasst, und wobei ein Signal von dem Amplifikationsnachweisteil eine Reaktionsumgebung identifiziert, in der ein gewünschtes Maß der Amplifikation erreicht worden ist.

8. Verfahren nach einem der Ansprüche 1 bis 7, wobei die Reaktionsumgebung keine anderen Genome als das Genom der einzelnen Zelle umfasst.

9. Verfahren nach einem der Ansprüche 1 bis 8, wobei im Wesentlichen alle Amplicons der Mehrzahl von Amplicons unverzweigt sind.

10. Verfahren nach einem der Ansprüche 1 bis 9, wobei die Mehrzahl von Amplicons nicht mehr als etwa 100 Picogramm bis etwa 10 Nanogramm DNA umfasst.

11. Verfahren nach einem der vorstehenden Ansprüche, wobei das Verfahren parallel an zwei oder mehr Genomen von zwei oder mehr einzelnen Zellen durchgeführt wird, wodurch zwei oder mehr verzerrungsfreie Amplifikationsbibliotheken parallel erzeugt werden.

12. Verfahren zur Erzeugung einer im Wesentlichen verzerrungsfreien Amplifikationsbibliothek eines Genoms durch Multiple-Strand-Displacement-Amplifikation (MDA), wobei das Verfahren folgendes umfasst:
- Applizieren einer Flüssigkeit, die verdünnte Ganzzellen oder Teile dieser umfasst, auf ein Substrat, das eine Reaktionsumgebung im Nanolitermaßstab umfasst, die ein Volumen von 1 Nanoliter bis 50 Nanolitern aufweist, wobei eine Wahrscheinlichkeit von wenigstens 99% besteht, dass die Reaktionsumgebung im Nanolitermaßstab nicht mehr als ein Genom umfasst, wobei ein Verhältnis der Nukleinsäuremenge des Genoms zu dem Volumen der Reaktionsumgebung im Nanolitermaßstab wenigstens 0,3 Megabasenpaare pro Nanoliter beträgt; und
- Inkontaktbringen der Reaktionsumgebung mit (a) Strand-Displacement-Polymerase und (b) einer Mehrzahl wahlfreier Multimere von DNA, und Amplifizieren des Genoms durch MDA bis sich zusätzliche Amplifikationszyklen nicht mehr in einer logarithmischen Phase befinden, wodurch eine im Wesentlichen verzerrungsfreie Amplifikation des Genoms erzeugt wird.

13. Verfahren nach Anspruch 12, ferner umfassend das Aufbauen einer Bibliothek, die eine Mehrzahl von Amplicons der im Wesentlichen verzerrungsfreien Amplifikation des Genoms umfasst.

14. Verfahren nach einem der Ansprüche 1 bis 11, wobei das Applizieren das gleichmäßige Ausbreiten der Mehrzahl von Zellen über eine Beschickungsfläche des Substrats umfasst, wobei wenigstens 99% der Mehrzahl von Reaktionsumgebungen im Nanolitermaßstab nicht mehr als eine Zelle je Näpfchen enthält.

## Revendications

1. Procédé de production d'une bibliothèque d'amplification pratiquement non biaisée d'un génome d'une cellule isolée, le procédé comprenant les étapes consistant à :
appliquer un liquide comprenant des cellules entières diluées ou des fractions de celles-ci et la cellule isolée à un substrat comprenant une pluralité d'environnements réactionnels à l'échelle du nanolitre ayant chacun un volume de 1 nanolitre à 50 nanolitres, la cellule isolée étant dans un environnement réactionnel à l'échelle du nanolitre de la pluralité d'environnements réactionnels, et au moins 95 % des environnements réactionnels à l'échelle du nanolitre ne comprenant aucun génome autre que le génome de la cellule isolée ;
amplifier, par amplification par déplacements multiples de brins, le génome de la cellule isolée dans l'environnement réactionnel à l'échelle du nanolitre, un rapport de la quantité d'acide nucléique du génome sur le volume de l'environnement réactionnel à l'échelle du nanolitre étant d'au moins 0,3 méga-paires de base par nanolitre, et l'environnement réactionnel à l'échelle du nanolitre étant conçu pour une amplification pratiquement non biaisée du génome, ladite amplification comprenant l'étape consistant à mettre en contact l'environnement réactionnel avec (a) une polymérase à déplacement de brins, et (b) une pluralité de multimères aléatoires d'ADN, ladite amplification étant réalisée jusqu'à ce que des cycles additionnels d'amplification ne soient plus dans une phase logarithmique, produisant ainsi une pluralité d'amplicons d'une amplification pratiquement non biaisée du génome ; et
construire une bibliothèque comprenant une pluralité d'amplicons de l'amplification pratiquement non biaisée du génome.

2. Procédé selon la revendication 1, l'environnement réactionnel à l'échelle du nanolitre étant conçu pour l'amplification d'au moins environ 90 % du génome à une couverture supérieure à 1x.

3. Procédé selon la revendication 1 ou 2, l'environnement réactionnel à l'échelle du nanolitre comprenant un volume inférieur ou égal à environ 20 nL.

4. Procédé selon l'une quelconque des revendications 1 à 3, comprenant en outre l'étape consistant à amplifier une pluralité de génomes de cellules isolées dans la pluralité d'environnements réactionnels à l'échelle du nanolitre sur un substrat unique, au moins 95 % des environnements réactionnels ne comprenant aucun génome autre qu'un génome d'une cellule isolée.

5. Procédé selon la revendication 4, comprenant en outre l'étape consistant à identifier un environnement réactionnel dans lequel un niveau souhaité d'amplification a été atteint, la bibliothèque étant construite à partir de l'environnement réactionnel dans lequel un niveau souhaité d'amplification a été atteint.

6. Procédé selon la revendication 4 ou 5, comprenant en outre l'étape consistant à construire une pluralité de bibliothèques à partir de la pluralité d'environnements réactionnels, le nombre de la pluralité de bibliothèques étant identique ou différent du nombre de la pluralité d'environnements réactionnels.

7. Procédé selon l'une quelconque des revendications 1 à 6, l'amplification du génome de la cellule isolée dans l'environnement réactionnel à l'échelle du nanolitre comprenant l'amplification en présence d'une fraction amplification-détection, et le signal provenant de la fraction amplification-détection identifiant un environnement réactionnel dans lequel un niveau souhaité d'amplification a été atteint.

8. Procédé selon l'une quelconque des revendications 1 à 7, l'environnement réactionnel ne comprenant pas des génomes autres que le génome de la cellule isolée.

9. Procédé selon l'une quelconque des revendications 1 à 8, pratiquement tous les amplicons de la pluralité d'amplicons étant non ramifiés.

10. Procédé selon l'une quelconque des revendications 1 à 9, la pluralité d'amplicons ne comprenant pas plus d'environ 100 picogrammes à environ 10 nanogrammes d'ADN.

11. Procédé selon l'une quelconque des revendications ci-dessus, le procédé étant effectué en parallèle sur deux génomes ou plus de deux cellules isolées ou plus, produisant ainsi deux bibliothèques d'amplification non biaisées ou plus en parallèle.

12. Procédé de production d'une bibliothèque d'amplification pratiquement non biaisée d'un génome par amplification par déplacements multiples de brin (MDA), le procédé comprenant les étapes consistant à :
appliquer un liquide comprenant des cellules entières diluées ou des fractions de celles-ci à un substrat comprenant un environnement réactionnel à l'échelle du nanolitre ayant un volume de 1 nanolitre à 50 nanolitres, avec au moins 99 % de probabilité que l'environnement réactionnel à l'échelle du nanolitre ne comprenne pas plus d'un génome, un rapport de la quantité d'acide nucléique du génome sur le volume de l'environnement réactionnel à l'échelle du nanolitre étant d'au moins 0,3 méga-paires de base par nanolitre ; et
mettre en contact l'environnement réactionnel à l'échelle du nanolitre avec (a) une polymérase à déplacement de brins, et (b) une pluralité de multimères aléatoires d'ADN, et amplifier le génome par MDA jusqu'à ce que des cycles additionnels d'amplification ne soient plus dans une phase logarithmique, produisant ainsi une amplification pratiquement non biaisée du génome.

13. Procédé selon la revendication 12, comprenant en outre l'étape consistant à construire une bibliothèque comprenant une pluralité d'amplicons de l'amplification pratiquement non biaisée du génome.

14. Procédé selon l'une quelconque des revendications 1 à 11, ladite application comprenant l'étape consistant à étaler uniformément la pluralité de cellules sur une zone de charge du substrat, de sorte qu'au moins 99 % de la pluralité d'environnements réactionnels à l'échelle du nanolitre ne contiennent pas plus d'une cellule par puits.
